# EUROPEAN PATENT SPECIFICATION

(11) **EP 1 904 049 B1**
(45) Date of publication and mention of the grant of the patent: **07.12.2016**
(21) Application number: 06774537.2
(22) Date of filing: 07.07.2006
(51) Int. Cl.: A61K 31/202, A61P 25/28, A61K 31/22

(54) **POLYUNSATURATED FATTY ACIDS FOR TREATMENT OF DEMENTIA AND PRE-DEMENTIA-RELATED CONDITIONS**
MEHRFACH UNGESÄTTIGTE FETTSÄUREN ZUR BEHANDLUNG VON DEMENZ UND PRÄ-DEMENZ-ERKRANKUNGEN
ACIDES GRAS POLYINSATURES POUR LE TRAITEMENT DE LA DEMENCE ET D'ETATS PREDEMENTIELS

(30) Priority: 08.07.2005 US 697911 P; 02.03.2006 US 779145 P
(43) Date of publication of application: 02.04.2008
(73) Proprietor: DSM IP Assets B.V., 6411 TE Heerlen (NL)
(72) Inventor: ELLIS, Lorie, A., Bel Air, MD 21014 (US)
(74) Representative: D Young & Co LLP
(86) International application number: PCT/US2006/026331
(87) International publication number: WO 2007/008586

(56) References cited:
- EP-A2- 0 342 795
- WO-A1-98/03671
- US-A- 5 403 826
- LIM G P ET AL: "Diets enriched with polyunsaturated DHA ( docosahexanoic acid ) can lower amyloid levels and plaque burden in an Alzheimer's disease mouse model.", SOCIETY FOR NEUROSCIENCE ABSTRACT VIEWER AND ITINERARY PLANNER, vol. 2003, 2003, XP8152384, & 33RD ANNUAL MEETING OF THE SOCIETY OF NEUROSCIENCE; NEW ORLEANS, LA, USA; NOVEMBER 08-12, 2003
- COLE G M ET AL: "SUPPRESSION OF ALZHEIMER'S DISEASE ( AD ) PATHOGENESIS BY DIETARY LIPIDS AND NSAIDS", ABSTRACTS OF THE ANNUAL MEETING OF THE SOCIETY FOR NEUROSCIENCE, SOCIETY FOR NEUROSCIENCE, WASHINGTON, DC, US, vol. 32, 7 November 2002 (2002-11-07), XP009130008, ISSN: 0190-5295
- DE WILDE M C ET AL: "The omega - 3 fatty acid docosahexaenoic acid ( DHA ) inhibits the formation of beta amyloid in CHO7PA2 cells.", SOCIETY FOR NEUROSCIENCE ABSTRACT VIEWER AND ITINERARY PLANNER, vol. 2003, 2003, XP8152453, & 33RD ANNUAL MEETING OF THE SOCIETY OF NEUROSCIENCE; NEW ORLEANS, LA, USA; NOVEMBER 08-12, 2003
- MORIHARA T ET AL: "Dietary fat regulation of inflammatory gene expression in an Alzheimer model ( TG2576 ) .", SOCIETY FOR NEUROSCIENCE ABSTRACT VIEWER AND ITINERARY PLANNER, vol. 2003, 2003, XP8152445, & 33RD ANNUAL MEETING OF THE SOCIETY OF NEUROSCIENCE; NEW ORLEANS, LA, USA; NOVEMBER 08-12, 2003
- MORIMOTO MIO ET AL: "The involvement of the arachidonic acid cascade and inflammatory cytokines in impairment of working memory induced by a beta-amyloid precursor protein (CT105).", JOURNAL OF PHARMACOLOGICAL SCIENCES, vol. 94, no. Supplement 1, 2004, page 78P, XP8152424, & 77TH ANNUAL MEETING OF THE JAPANESE PHARMACOLOGICAL SOCIETY; OSAKA, JAPAN; MARCH 08-10, 2004 ISSN: 1347-8613
- LIM ET AL.: 'Diets enriched with polyunsaturated DHA (docosahexanoic acid) can lower amyloid levels and plaque burden in an Alzheimer's disease mouse model' SOCIETY FOR NEUROSCIENCE ABSTRACT VIEWER AND ITINERARY PLANNER vol. 2003, 2003, page ABSTR. NO. 201.25, XP002369925
- LIM SUN-YOUNG ET AL: "An extraordinary degree of structural specificity is required in neural phospholipids for optimal brain function: n-6 docosapentaenoic acid substitution for docosahexaenoic acid leads to a loss in spatial task performance", JOURNAL OF NEUROCHEMISTRY, vol. 95, no. 3, November 2005 (2005-11), pages 848-857, XP55117566, ISSN: 0022-3042

## Description

### Field of the Invention

The present invention generally relates to compositions for treating or preventing dementia pre-dementia and/or symptoms or characteristics of such conditions.

### Background of the Invention

A decline in memory and cognitive function is considered to be a normal consequence of aging in humans. Age-related cognitive decline is a term used to describe objective memory decline in the elderly who have cognitive functioning that is normal relative to their age peers. Age-related cognitive decline is different from Mild Cognitive Impairment (MCI) that is more severe or consistent, and may indicate the early stages of a condition such as dementia (APA Presidential Task Force on the Assessment of Age-Consistent Memory Decline and Dementia, February 1998). Given the high prevalence of age-related cognitive decline, memory loss is a prominent health concern for many individuals age 55 and older.

Dementia is characterized by loss of integrated central nervous system functions, resulting in the inability to understand simple concepts or instructions, to store and retrieve information into memory, and in behavioral and personality changes. The most commonly used criteria for diagnoses of dementia are the DSM-IV (Diagnostic and Statistical Manual for Mental Disorders, American Psychiatric Association). Diagnostic features of dementia according to the DSM-IV include memory impairment and at least one of the following: language impairment (aphasia), lost ability to execute learned motor functions (apraxia), inability to recognize familiar objects (agnosia), or disturbances in executive functioning or decision making.

The most prevalent forms of dementia in the United States are Alzheimer's Disease (40 to 60% of diagnoses); Vascular Dementia (10 to 20% of all diagnoses); Mixed Dementia (10% of all diagnoses); Dementia with Lewy Bodies (10% of all diagnoses). Secondary dementias caused by drugs, delirium, or depression represent 5% or less of all dementia diagnoses in the United States.

Alzheimer's disease (AD) is classified as dementia with neurodegeneration, and is prevalent worldwide. Diagnosis of AD is confirmed postmortem by the accumulation of amyloid plaques and neurofibrillary tangles, synaptic loss and neurodegeneration in several regions of the brain and enlargement of brain ventricles. Senile dementia itself refers to all dementia in the population age 65 and over and includes AD. However, the pathogenic mechanisms responsible for the development of AD are poorly understood.

Histologically, the key neuropathological findings in AD include: increased intraneuronal amyloid peptide, amyloid plaques, neurofibrillary tangles, synaptic loss, and neuronal cell death. Amyloid plaque load is a predictor of AD severity, although increased plaque load is not strongly correlated with impairment of cognitive function. Morris and Price (2001) suggest that widespread amyloid plaques in the neocortex best distinguish very early stage AD. Other AD lesions, including increased formation of neurofibrillary tangles and neuronal degeneration, appear to result from the amyloid-initiated pathologic process, although they may have a more immediate effect on expression and severity of dementia.

Fish consumption in individuals, and particularly the elderly, has been linked with improved cognitive performance, reduced display of aggression and reduced risk of Alzheimer's Disease and dementia. Supplementation with a combination of docosahexaenoic acid (DHA) and eicosapentaenoic acid (EPA) has been shown to improve cognitive performance and visual acuity in subjects with and without dementia (Suzuki et al (2001)). In addition, studies in a transgenic mouse model of Alzheimer's disease, where the mice have a mutation in the gene encoding amyloid precursor protein (APP), indicated that DHA reduces amyloid levels and plaque burden. However, the mechanism of action of the effects of fish consumption or of DHA or EPA consumption has not been determined prior to the present invention, particularly with regard to the pathophysiology of Alzheimer's Disease.

Treatment or prevention of neurological diseases or injuries traditionally focuses on a pharmaceutical approach. For example, neuropsychiatric or neurodegenerative drugs are continually being developed which alleviate symptoms, but fail to alleviate the inherent cause of the neurological problem. Thus, there is a further need in the art for novel therapeutic strategies for the treatment of neurological disorders that are manifested in dementia, such as AD. Additionally, although evidence exists that fish consumption and/or supplementation with DHA and EPA may reduce symptoms related to AD, there is a need in the art to identify the mechanism of action of such therapy, so that improved formulations and protocols can be developed and so that individuals at risk of developing AD can be easily identified and treated at a timepoint when the treatment will be most effective, particularly prior to the onset of clinical symptoms.

Lim P, et al "Diets enriched with polyunsaturated DHA can lower amyloid levels and plaque burden in Alzheimer's disease mouse model", Society for Neuroscience Abstract Viewer and Itinery planner, vol. 2003, 2003, and 33rd Annual Meeting of the Society of Neuroscience; New Orleans, LA, USA; November, 2003 discloses that diets enriched with DHA can lower amyloid levels.

EP0342795A2 discloses the use of DHA for improving brain function

Cole, G. M. et al, Abstracts of the Annual Meeting of the Society fo Neuroscience, Washington D.C., US, vol. 32, 7 November 20012, discusses suppression of Alzheimer's disease pathogenesis by dietary lipids and NSAIDs.

De Wilde, M. C. et al "The omega-3 fatty acid DHA inhibits the formation of beta amyloid in CHO7PA2 cells", Society for Neuroscience Abstract Viewer and Itinery planner, vol. 2003, 2003, and 33rd Annual Meeting of the Society of Neuroscience; New Orleans, LA, USA; November, 2003 discloses that DHA can inhibit formation of beta amyloid levels in CHO7PA2 cells.

Morihara, T. et al, Society for Neuroscience Abstract Viewer and Itinery planner, vol. 2003, 2003, and 33rd Annual Meeting of the Society of Neuroscience; New Orleans, LA, USA; November, 2003 discloses dietary fat regulation of inflammatory gene expression in an Alzheimer model.

Morimoto Mio et al, Journal of pharmacological sciences, vol. 94, no. Supplement 1, 2004, page 78P and 77th Annual meeting of the Japanese Pharmacological Society, Osaka, Japan, 2004 describes the involvement of the arachidonic acid cascade and inflammatory cytokines in impairment working memory induced by a beta-amyloid precursor protein.

### Summary of the Invention

The present invention provides use of a composition comprising docosahexaenoic acid (DHA) and docosapentaenoic acid n-6 (DPAn-6), wherein the ratio of DHA to DPAn-6 is from about 1:1 to about 10:1, in the preparation of a medicament formulated for oral administration for treating dementia or pre-dementia.

The present invention also provides a pharmaceutical composition comprising docosahexaenoic acid (DHA) and docosapentaenoic acid n-6 (DPAn-6) in a ratio of from about 1:1 to about 10:1 in an oral dosage for the treatment of dementia or pre-dementia in a human.

Described is a method to reduce the level of amyloid β (Aβ) peptide in an individual. The described method includes administering to an individual at least one polyunsaturated fatty acid (PUFA) or a precursor or source thereof to reduce the level of Aβ peptide in the individual, wherein the PUFA is selected from: docosahexaenoic acid (DHA); docosapentaenoic acid (DPAn-6); a combination of DHA and DPAn-6; a combination of DHA and arachidonic acid (ARA); and a combination of DHA, DPAn-6 and ARA. In one aspect, the Aβ peptide is soluble Aβ peptide.

Yet another described method relates to a method to reduce the level of tau protein in an individual. The described method includes administering to an individual at least one polyunsaturated fatty acid (PUFA) or a precursor or source thereof to reduce the level of tau protein in the individual, wherein the PUFA is selected from: docosahexaenoic acid (DHA); docosapentaenoic acid (DPAn-6); a combination of DHA and DPAn-6; a combination of DHA and arachidonic acid (ARA); and a combination of DHA, DPAn-6 and ARA. In one aspect, the tau protein is phosphorylated tau protein.

Another described method relates to a method to reduce the level of presenilin-1 (PS1) protein in an individual. The described method includes administering to an individual at least one polyunsaturated fatty acid (PUFA) or a precursor or source thereof to reduce the level of PS1 protein in the individual, wherein the PUFA is selected from: docosahexaenoic acid (DHA); docosapentaenoic acid (DPAn-6); a combination of DHA and DPAn-6; a combination of DHA and arachidonic acid (ARA); and a combination of DHA, DPAn-6 and ARA.

Another described method relates to a method to delay the onset of or reduce the severity of synaptic dysfunction in an individual. The described method includes administering to the individual DHA, DPAn-6, or in preferred embodiments, a combination of polyunsaturated fatty acids (PUFAs) or precursors or sources thereof to delay the onset of or reduce the severity of synaptic dysfunction in the individual, wherein the combination of PUFAs is selected from: a combination of DPAn-6 and DHA, a combination of ARA and DHA, and a combination of DPAn-6, ARA and DHA.

Yet another described method relates to a method to delay the onset of or reduce the severity of dementia in an individual. The described method includes administering to an individual DHA, DPAn-6, or in preferred embodiments, a combination of polyunsaturated fatty acids (PUFAs) or precursors or sources thereof to delay the onset of or reduce the severity of dementia in the individual, wherein the combination of PUFAs is selected from: a combination of DPAn-6 and DHA, a combination of ARA and DHA, and a combination of DPAn-6, ARA and DHA.

Another described method relates to a method to treat or prevent a disorder associated with increased amounts or expression of or dysfunction of amyloid β (Aβ) peptide, presenilin-1 (PS1) protein, phosphorylated tau protein, or tau protein. The described method includes the steps of: (a) identifying an individual having increased amount, expression, or biological activity of a biomarker selected from Aβ peptide, PS 1 protein, phosphorylated tau protein, tau protein, and combinations thereof, as compared to the amount, expression or biological activity of the biomarker in a negative control individual; and (b) administering to the individual at least one polyunsaturated fatty acid (PUFA) or a precursor or source thereof to reduce the amount, expression or biological activity of the Aβ peptide, PS 1 protein, phosphorylated tau protein, or tau protein, wherein the PUFA is selected from: docosahexaenoic acid (DHA); docosapentaenoic acid (DPAn-6); a combination of DHA and DPAn-6; a combination of DHA and arachidonic acid (ARA); and a combination of DHA, DPAn-6 and ARA.

Another described method relates to a method to treat or prevent a disorder associated with decreased amounts of omega-3 or omega-6 polyunsaturated fatty acid (PUFA) or a precursor or source thereof. The described method includes the steps of: (a) identifying an individual with decreased amounts of omega-3 or omega-6 polyunsaturated fatty acid (PUFA) or a precursor or source thereof; and (b) administering to the individual a combination of polyunsaturated fatty acids (PUFAs) or precursors or sources thereof to compensate for the effects of the decreased amounts of omega-3 or omega-6 polyunsaturated fatty acid (PUFA) or a precursor or source thereof, wherein the combination of PUFAs is selected from: a combination of DPAn-6 and DHA, a combination of ARA and DHA, and a combination of DPAn-6, ARA and DHA.

Another described method relates to a method to delay the onset of or reduce the severity of a decline in brain function in an individual. The described method includes administering to the individual a DHA, DPAn-6, or in preferred embodiments, a combination of polyunsaturated fatty acids (PUFAs) or precursors or sources thereof, to delay the onset of or reduce the severity of a decline in the brain function in the individual, wherein the combination of PUFAs is selected from: DPAn-6, a combination of DPAn-6 and DHA, a combination of ARA and DHA, and a combination of DPAn-6, ARA and DHA. In one described aspect, brain function is measured by a method selected from neuropsychological or cognitive tests, brain imaging methods (PET, SPECT, CT, MRI, fMRI), and electroencephalography (EEG).

Another described method relates to a method to delay or reduce the severity of demyelination in an individual. The described method includes administering to the individual a combination of polyunsaturated fatty acids (PUFAs) or precursors or sources thereof, to delay or reduce the severity of demyelination in the individual, wherein the combination of PUFAs is selected from: a combination of DPAn-6 and DHA, a combination of ARA and DHA, and a combination of DPAn-6, ARA and DHA.

Yet another describe method relates to a method to delay the onset of or reduce the severity of neurofibrillary tangles associated with Alzheimer's Disease in an individual. The described method includes administering to the individual DHA, DPAn-6, or in preferred embodiments, a combination of polyunsaturated fatty acids (PUFAs) or precursors or sources thereof, to delay the onset of or reduce the severity of neurofibrillary tangles in the individual, wherein the combination of PUFAs is selected from: a combination of DPAn-6 and DHA, a combination of ARA and DHA, and a combination of DPAn-6, ARA and DHA.

Another described method relates to a method to stabilize or normalize theta wave activity or to reduce or prevent the development of abnormal theta wave activity in an individual. The described method includes the steps of: (a) identifying an individual that has or is predicted to develop abnormal theta wave activity; and (b) administering to the individual at least one polyunsaturated fatty acid (PUFA) or a precursor or source thereof to stabilize or normalize theta wave activity or to prevent or reduce the development of abnormal theta wave activity in an individual, wherein the PUFA is selected from: docosahexaenoic acid (DHA); docosapentaenoic acid (DPAn-6); a combination of DHA and DPAn-6; a combination of DHA and arachidonic acid (ARA); and a combination of DHA, DPAn-6 and ARA.

In embodiments of the invention, the individual is identified as being susceptible to or having dementia or pre-dementia. In one aspect, the dementia is Alzheimer's disease. For example, in one aspect, the individual is identified as having or being susceptible to dementia or pre-dementia by measurement of a biological marker, a family history showing dementia, mild cognitive impairment, or age-related cognitive decline. The biological marker can include, but is not limited to, APP, Aβ peptide, tau protein, phosphorylated tau protein, PS1, and an omega-3 or an omega-6 polyunsaturated fatty acid (PUFA), or a precursor or source thereof. In one aspect, an amount, expression or biological activity of the biological marker is measured in a biological sample from the individual. The biological sample can include, but is not limited to, a cell sample, a tissue sample, and a bodily fluid sample, and particularly preferred samples include cerebrospinal fluid or a blood sample.

In one described method, prior to the step of administering, the described method can include measuring an amount, expression or a biological activity of a biomarker selected from APP, Aβ peptide, tau protein, phosphorylated tau protein, and PS 1 protein in a biological sample from the individual. In one described method, the described method can further include comparing the amount, expression or biological activity of the biomarker in the individual sample to a baseline amount, expression or biological activity of the biomarker in a sample of the same type, wherein an increase in the amount, expression or biological activity of the biomarker in the individual sample, as compared to the baseline amount, expression or biological activity, indicates that the individual is at risk of developing or has dementia.

In one described method, prior to the step of administering, the described method can include measuring an amount or biological activity of omega-3 or an omega-6 polyunsaturated fatty acid (PUFA), or a precursor or source thereof in a biological sample from the individual. In one described method, the method can further include comparing an amount or biological activity of omega-3 or an omega-6 polyunsaturated fatty acid (PUFA), or a precursor or source thereof in the individual sample to a baseline amount or biological activity of the omega-3 or an omega-6 polyunsaturated fatty acid (PUFA), or a precursor or source thereof in a sample of the same type, wherein a change in the amount of the omega-3 or an omega-6 polyunsaturated fatty acid (PUFA), or a precursor or source thereof in the individual sample as compared to the baseline amount indicates that the individual is at risk of developing or has dementia.

The step of measuring an amount or activity of a biomarker or omega-3 or omega-6 PUFA can include, but is not limited to, Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorption/ionization time-of flight (MALDI-TOF) mass spectrometry, microcytometry, microarray, microscopy, fluorescence activated cell sorting (FACS), flow cytometry, capillary electrophoresis, protein microchip or microarray, fatty acid methyl esterification/gas chromatography, thin-layer chromatography, Gas chromatography/mass spectroscopy, and liquid chromatography/mass spectroscopy.

Any of the above-described methods can further include monitoring the efficacy of the administration of the PUFA on Aβ peptide, tau protein, phosphorylated tau protein, or PS1 protein levels in the individual at least one time subsequent to the step of administering. Similarly, any of the above-described methods can further include monitoring the efficacy of the administration of the PUFA on omega-3 or omega-6 polyunsaturated fatty acid (PUFA) levels in the individual at least one time subsequent to the step of administering. Based on these results of monitoring, the method can further include adjusting the administration of the PUFA to the individual in subsequent treatments.

In any of the above-described embodiments of the invention, the PUFA can include an oil comprising 30%, 40%, 50%, 60%, 70%, 80%, or more of the PUFA, wherein the PUFA is in a chemical form selected from triglyceride form, triglyceride oil comprising the PUFA, phospholipids comprising the PUFA, a combination of protein and phospholipids comprising the PUFA, dried marine microalgae, sphingolipids comprising the PUFA, esters, as a free fatty acid, as a conjugate of the PUFA with another bioactive molecule, and combinations thereof.

In the present invention the PUFA comprises DPAn-6 and DHA, and the ratio of DPAn-6 to DHA is from about 1:1 to about 1:10. In any of the above-described embodiments of the invention, the PUFA can be administered, in one aspect, in an amount of from about 50 mg to about 20,000 mg per day. In another aspect, the PUFA can be administered in an amount of from about 0.025 mg per day to about 15 g per day. In another aspect, the PUFA can be administered in an amount of from about 0.05 mg/kg body weight per day to about 275 mg/kg body weight per day.

In any of the above-described embodiments of the invention, the source of the DHA and DPAn-6 can include, but is not limited to, fish oil, marine microalgae, plant oil, and combinations thereof.

Yet another embodiment of the invention relates to a pharmaceutical composition comprising DHA andDPAn-6 for treatment or prevention of dementia in an individual that has or is at risk of developing dementia. The combination of PUFAs comprises DPAn-6 and DHA. In one aspect, the combination of PUFAs comprises an oil comprising 30% or more or up to 80% or more of said PUFA, wherein the PUFA is in a chemical form selected from triglyceride form, triglyceride oil comprising the PUFA, phospholipids comprising the PUFA, a combination of protein and phospholipids comprising the PUFA, dried marine microalgae, sphingolipids comprising the PUFA, esters, as a free fatty acid, as a conjugate of the PUFA with another bioactive molecule, and combinations thereof. In one aspect, the source of the PUFA is selected from: fish oil, marine algae, plant oil, and combinations thereof. In one aspect, the PUFA is provided in a formulation selected from: chewable tablets, quick dissolve tablets, effervescent tablets, reconstitutable powders, elixirs, liquids, solutions, suspensions, emulsions, tablets, multi-layer tablets, bi-layer tablets, capsules, soft gelatin capsules, hard gelatin capsules, caplets, lozenges, chewable lozenges, beads, powders, granules, particles, microparticles, dispersible granules, cachets, douches, suppositories, creams, topicals, inhalants, aerosol inhalants, patches, particle inhalants, implants, depot implants, ingestibles, injectables, infusions, health bars, confections, cereals, cereal coatings, foods, nutritive foods, functional foods and combinations thereof. In one aspect, the PUFA in the formulation is provided in a form selected from: a highly purified algal oil comprising the PUFA, triglyceride oil comprising the PUFA, phospholipids comprising the PUFA, a combination of protein and phospholipids comprising the PUFA, dried marine microalgae comprising the PUFA, sphingolipids comprising the PUFA, esters of the PUFA, free fatty acid, a conjugate of the PUFA with another bioactive molecule, and combinations thereof.

A therapeutic compound that can be included in a composition of the invention can include, but is not limited to, a protein, an amino acid, a drug, and a carbohydrate. In one aspect, the therapeutic compound is selected from: tacrine; donepezil; rivastigmine; galantamine; memantine; neotropin; nootropics; alpha-tocopherol (vitamin E); selegeline; non-steroidal anti-inflammatory agents (NSAIDS); gingko biloba; estrogen; β-secretase inhibitors; vaccines; B complex vitamins; calcium channel blockers; HMG CoA reductase inhibitors; statins; policosanols; fibrates; clioquinol; curcumin; lignans; phytoestrogens; phytosterols; niacin; and vitamin supplements.

Another embodiment of the invention is the use of a composition comprising docosahexaenoic acid (DHA) and docosapentaenoic acid n-6 (DPAn-6), wherein the ratio of DHA to DPAn-6 is from about 1:1 to about 10:1, in the preparation of a medicament formulated for oral administration for treating dementia or pre-dementia. Described is reducing the level of amyloid β (Aβ) peptide in an individual; reducing the level of tau protein in an individual; reducing the level of presenilin-1 (PS1) protein in an individual; treating or preventing a disorder associated with increased amounts or expression of or dysfunction of amyloid β (Aβ) peptide, presenilin-1 (PS 1) protein, phosphorylated tau protein, or tau protein; and/or stabilizing or normalizing theta wave activity or reducing or preventing the development of abnormal theta wave activity in an individual.

Yet another embodiment of the invention relates to a pharmaceutical composition comprising docosahexaenoic acid (DHA) and docosapentaenoic acid n-6 (DPAn-6) in a ratio of from about 1:1 to about 10:1 in an oral dosage for the treatment of dementia or pre-dementia in a human. Described is a composition for delaying the onset of or reduce the severity of synaptic dysfunction in an individual; delaying the onset of or reduce the severity of dementia in an individual; treating or preventing a disorder associated with decreased amounts of omega-3 or omega-6 polyunsaturated fatty acid (PUFA); delaying the onset of or reducing the severity of a decline in brain function in an individual; delaying or reducing the severity of demyelination in an individual; and/or delaying the onset of or reducing the severity of neurofibrillary tangles associated with Alzheimer's Disease in an individual.

### Brief Description of the Figures

Figs. 1A-1C show whole brain fatty acid profiles in 3xTg-AD mice after dietary treatment for 3 months (Fig. 1A; n=6), 6 months (Fig. 1B; n=6), or 9 months (Fig. 1C; n=6).
Figs. 2A-2C show red blood cell fatty acid profiles in 3xTg-AD mice after dietary treatment for 3 months (Fig. 2A), 6 months (Fig. 2B), or 9 months (Fig. 2C).
Figs. 3A-3C show brain phosphatidylcholine (PC) profiles in 3xTg-AD mice after dietary treatment for 3 months (Fig. 3A), 6 months (Fig. 3B), or 9 months (Fig. 3C).
Figs. 4A-4C show brain phosphatidylethanolamine (PE) profiles in 3xTg-AD mice after dietary treatment for 3 months (Fig. 4A), 6 months (Fig. 4B), or 9 months (Fig. 4C).
Figs. 5A-5C show brain phosphatidylserine (PS) profiles in 3xTg-AD mice after dietary treatment for 3 months (Fig. 5A), 6 months (Fig. 5B), or 9 months (Fig. 5C).
Figs 6A-6F show soluble (Figs. 6A, 6C, 6E) and insoluble (Figs. 6B, 6D, F) Aβ levels in 3xTg-AD mice following DHA dietary treatment for 3 months (Figs. 6A and 6B; n=6), 6 months (Figs. 6C and 6D; n=6) and 9 months (Figs. 6E and 6F; n=6).
Figs. 6G-6J are digital images showing that DAB staining with 6E10 shows Aβ-like immunoreactivity in 40 µM sections from mice treated for 3 months with control diet (Fig. 6G), DHA/DPA diet (Fig. 6H), DHA diet (comparative) (Fig. 6I), and DHA/ARA diet (comparative) (Fig. 6J).
Figs 7A-7D show steady state levels (Figs. 7A and 7C) and quantification of the protein blots normalized to β-actin levels as a loading control (Figs. 7B and 7D) of APP fragments (Figs. 7A and 7B) and IDE (Figs. 7C and 7D) in 3xTg-AD mice following DHA dietary treatment.
Figs. 8A-8B show steady state levels of ADAM10 and BACE (Fig. 8A) and quantification of the protein blots normalized to β-actin levels as a loading control (Fig. 8B) in 3xTg-AD mice following DHA dietary treatment.
Figs. 8C-8D show steady state levels of presenilin 1 and nicastrin (Fig. 8C) and quantification of the protein blots normalized to β-actin levels as a loading control (Fig. 8D) in 3xTg-AD mice following DHA dietary treatment.
Fig. 8E shows that DHA significantly reduced presenilin 1 mRNA (*, p<0.05) in SHSY5Y cells treated for 48 hours with either 0.3 µg/ml DHA complexed 3:1 to BSA (n=3), or with the equivalent BSA alone (n=3).
Figs. 9A-9F shows the Tau steady state levels (Figs. 9A, 9C and 9E) and quantification of the protein blots normalized to β-actin levels as a loading control (Figs. 9B, 9D and 9F) in 3xTg-AD mice following DHA dietary treatment for 3 months (Figs. 9A and 9B), 6 months (Figs. 9C and 9D) and 9 months (Figs. 9E and 9F).
Figs. 10A-10D are digitized images showing conformationally altered Tau immunoreactivity in 3xTg-AD mice following dietary treatment for 3 months (Fig. 10A = control diet, Fig. 10B = DHA/DPA diet, Fig. 10C = DHA diet (comparative), and Fig. 10D = DHA/arachidonic acid (comparative)).
Fig. 10E is a digitized image of an immunoblot showing phosphorylated Tau after 9 months of dietary treatment.

### Detailed Description of the Invention

The present invention provides use of a composition comprising docosahexaenoic acid (DHA) and docosapentaenoic acid n-6 (DPAn-6), wherein the ratio of DHA to DPAn-6 is from about 1:1 to about 10:1, in the preparation of a medicament formulated for oral administration for treating dementia or pre-dementia.

The present invention also provides a pharmaceutical composition comprising docosahexaenoic acid (DHA) and docosapentaenoic acid n-6 (DPAn-6) in a ratio of from about 1:1 to about 10:1 in an oral dosage for the treatment of dementia or pre-dementia in a human.

The present invention generally relates to the use of polyunsaturated fatty acid (PUFA) supplementation (DHA and DPAn-6) to delay the onset of and/or reduce the severity of and/or symptoms of dementia or pre-dementia, including Alzheimer's disease (AD). Described are methods to reduce the level of amyloid-β (soluble or insoluble), tau protein, tau phosphorylated protein and/or presenilin-1 (PS1) protein in the neural tissue of an individual; to methods to delay or reduce the severity of synaptic dysfunction, decline in brain function, demyelination, formation of neurofibrillary tangles, and/or dementia in an individual; and to methods to treat or prevent a disorder associated with increased amounts of amyloid-β, tau protein (soluble or insoluble), phosphorylated tau protein and/or presenilin-1 (PS1) protein, or with decreased amounts of omega-3 and/or omega-6 PUFAs. Also described is a method to stabilize or normalize theta wave activity or reduce or prevent the development of abnormal theta wave activity in an individual. The medical uses of the present invention comprise administering to the individual a combination of DPAn-6 and DHA. The PUFA comprises DHA and DPAn-6 in combination with each other, and optionally in combination with other PUFAs.

According to the present invention, dementia is characterized by loss of integrated central nervous system functions resulting in the inability to understand simple concepts or instructions, to store and retrieve information into memory, and in behavioral and personality changes. The most commonly used criteria for diagnoses of dementia are the DSM-IV (Diagnostic and Statistical Manual for Mental Disorders, American Psychiatric Association). Diagnostic features of dementia according to the DSM-IV include memory impairment and at least one of the following: language impairment (aphasia), lost ability to execute learned motor functions (apraxia), inability to recognize familiar objects (agnosia), or disturbances in executive functioning or decision making. Alzheimer's disease (AD) is the most prevalent form of dementia.

Recently a mouse triple transgenic model of Alzheimer's disease has been developed and reported by a group at the University of California at Irvine (Oddo et al., 2003). These mice contain PS1_{M146V}, APP_{Swe}, and tau_{P301L} transgenes. Presenilin-1 (PS1) is a protein produced in brain cells, and is linked to increased amounts of Aβ peptides. Inherited mutations of the PS1 gene on chromosome 14 are a cause of Familial Alzheimer's Disease. Amyloid Precursor Protein (APP) is a protein of uncertain function that is normally found in the brain. In Alzheimer's disease, APP is abnormally degraded resulting in the formation of amyloid. Tau, a highly asymmetric and heat-stable protein, is expressed mainly in the brain, where it regulates the orientation and stability of microtubules in neurons, astrocytes and oligodendrocytes. These triple-transgenic mice develop both plaque and tangle pathology in Alzheimer's-relevant brain regions (hippocampus, amygdala, and cerebral cortex). They develop extracellular Aβ deposits prior to tangle formation, consistent with the amyloid cascade hypothesis. The mice exhibit synaptic dysfunction as early as six months of age. Although no extracellular Aβ deposits are localized to the hippocampal region at this age, intracellular accumulation of the Aβ peptide, including the most pathogenic Aβ₄₂ peptide, has been demonstrated at this age. The findings of this model suggest that intraneuronal Aβ underlies the observed synaptic dysfunction. Tangle formation typically initiates in limbic brain structures and progresses to cortical regions in human individuals, which is the pattern observed in this mouse model.

The present inventor has used this mouse model that closely mimics human AD to determine the mechanism of action of PUFAs on the development and progression of AD, and to develop novel therapeutic strategies and compositions for the prevention and treatment of AD. The inventor provided groups of these mice with diets that included a significant dietary source of one or more PUFAs. The diets included a diet enriched in DHA, a diet enriched in both DHA and DPAn-6, and a diet enriched in DHA and ARA. The inventor has surprisingly discovered that administration of PUFAs actually decreases the amount of soluble Aβ, decreases the amount of total tau protein, and decreases the amount of presenilin-1 in this animal model. In particular, the inventor has discovered that DHA-containing diets reduce soluble Aβ levels, although the effects of the DHA are diminished over time, particularly in individuals where adrenic acid or arachidonic acid levels are elevated, or in individuals in which DPAn-6 levels are reduced. Diets containing DHA alone were the most effective over time, but the combination of DHA and DPAn-6 was also effective at earlier timepoints, and the combination of DHA and ARA was also effective in early timepoints. Moreover, the inventor found that the combination of DHA and DPAn-6 delivers a surprising, unexpected benefit, as animals that were provided with sufficient dietary preformed DHA in combination with DPAn-6 had significantly increased DPAn-6DPAn-6 levels in tissue as compared to animals fed DHA alone. Animals with higher blood DHA and ARA levels tended to have greater reduction in presenilin-1, the protein associated with cleavage of APP into toxic amyloid peptides, although diets containing DHA alone or combinations of DHA and DPAn-6 also reduced presenilin-1 levels. The inventor also found that DHA-containing diets reduced total tau levels, including diets containing DHA alone, DHA and DPAn-6 or DHA and ARA, although the effects of combination diets waned over time, such that only diets containing DHA as the predominant PUFA resulted in a reduced total tau level. However, with respect to phosphorylated tau, diets containing DHA or DHA and DPAn-6 both significantly reduced phosphorylated tau, even at later timepoints, with the combination of DHA and DPAn-6 being as effective and trending toward more effective than the diet enriched only in DHA. Taken together, the inventor's results indicate that diets containing DHA (not in accordance with the invention) or DHA and DPAn-6 (in accordance with the invention) are effective at reducing AD pathologies, and that the most successful formulation overall is a diet containing DHA alone as the predominant PUFA, although diets with DHA and DPAn-6 are also effective with respect to reducing at least phosphorylated tau. More particularly, the present inventor has discovered that PUFAs have different effects on the pathology of cognitive disorders such as AD, so that patients can be targeted appropriately, and/or so that PUFAs can be administered in preferred doses or timepoints to more efficiently treat the disease. Specifically, the present inventor has discovered that DHA reduces amyloid, which is believed to be an earlier pathology, while DPAn-6 is additionally effective in reducing tau and phospho-tau pathology, which are believed to be later biomarkers of pathology. Moreover, the beneficial effects observed from diets containing DPAn-6 were also surprising, since prior to the present invention, no role for DPAn-6 in improving a symptom or treating a characteristic of AD had been described. The inventor's results also provide powerful new markers for identifying subjects that are most likely to respond positively to PUFA supplementation or treatment at various timepoints in disease.

Also described is a method to reduce the level of Aβ peptide in an individual's neural tissue, comprising administering to an individual an amount of at least one omega-3 and/or omega-6 polyunsaturated fatty acid (PUFA), and/or a precursor or source thereof, to reduce the level of Aβ peptide in the individual. The Aβ peptide that is decreased may be soluble Aβ peptide. In certain examples, the PUFA comprises DHA, DPAn-6DPAn-6, a combination of DPAn-6 and DHA, a combination of DHA and ARA, or a combination of DPAn-6, ARA, and DHA. In one example, the PUFA comprises DHA. In one example, the PUFA comprises DPAn-6. In another example, the PUFA is a combination of DHA and DPAn-6.

In another example, described is a method to maintain the level of APP in an individual's neural tissue, or more preferably, to prevent or inhibit the cleavage of APP (regardless of the level) into Aβ peptide by presenilin-1, and particularly into the more toxic size forms of Aβ peptide. The described method comprises administering to an individual an amount of at least one omega-3 and/or omega-6 polyunsaturated fatty acid (PUFA), and/or a precursor or source thereof, to maintain the level of APP in the individual, or to prevent or inhibit the cleavage of APP into Aβ peptide by presenilin-1 into the more toxic size forms of Aβ peptide. In certain examples, the PUFA comprises DHA, DPAn-6, a combination of DPAn-6 and DHA, or a combination of DPAn-6, ARA, and DHA. In one examples, the PUFA comprises DHA. In one embodiment, the PUFA comprises DPAn-6. In another example, the PUFA is a combination of DHA and DPAn-6.

The main component of the extracellular amyloid plaques is the amyloid protein (Aβ), which is a 42 to 43 amino-acid peptide derived from proteolytic cleavage of the amyloid protein precursor (APP), a type I transmembrane glycoprotein of unknown function. Three distinct enzymes cleave APP to produce beta amyloid peptides that differ in size. Alpha-secretase cleaves from the C-terminal end of APP at a site within the Aβ peptide sequence (Aβ 16) generating an 83 amino acid C-terminal fragment and a 26 amino acid form of Aβ. Gamma-secretase subsequently cleaves the 83 amino acid peptide at the C-terminal end of the Aβ peptide, releasing a short 15 amino-acid peptide (p3) whose role in amyloidogenesis is not well defined. Beta-secretase, or BACE1, cuts on the N-terminal end of the Aβ peptide, while gamma-secretase, which may, in fact, be a presenilin, cuts on the C-terminal end of the Aβ peptide. Depending on exactly where gamma-secretase cleaves the APP in conjunction with beta-secretase, a 40 or 42 amino acid peptide is produced. The longer 42-43 amino acid peptide is believed to be a more pathogenic form of Aβ compared to the 15 or the 40 amino acid forms. Aβ-derived diffusible ligands (or ADDLs) are non-fibrillar, aggregating derivatives of Aβ 1-42 with reportedly greater neurotoxicity compared to large Aβ fibrils found within Alzheimer plaques. Aβ 1-42 aggregation into oligomeric ADDLs is increased in the presence of clusterin (Apo J), a protein component of senile plaques. Aβ 1-42+clusterin is highly toxic in PC12 cultured neurons.

The nucleotide sequence encoding human amyloid protein precursor (APP) has been determined, and the nucleotide and encoded amino acid sequences for the APP peptide can be found in public sequence databases, such as the National Center for Biotechnology Information (NCBI) database. For example, nucleotide and amino acid sequences for human APP peptide can be found in the NCBI database under Accession Nos. NM_000484 (variant 1), NM_201413 (variant 2), and NM_201414 (variant 3). Detection and/or measurement of Aβ peptide can be accomplished by methods known in the art and are discussed in detail below.

Also described is a method to reduce the level of tau protein in an individual's neural tissue, comprising administering to an individual an amount of at least one omega-3 and/or an omega-6 polyunsaturated fatty acid (PUFA), and/or a precursor or source thereof, to reduce the level of tau protein in the individual. In an example, the tau protein to reduce is a phosphorylated tau protein or a conformationally changed tau protein. In certain examples, the PUFA comprises DHA, DPAn-6, a combination of DPAn-6 and DHA, a combination of DHA and ARA, or a combination of DPAn-6, ARA, and DHA. In one example, the PUFA comprises DHA. In one example, the PUFA comprises DPAn-6. The PUFA is a combination of DHA and DPAn-6.

Tau protein, and particularly phosphorylated tau, is the predominant protein found in Neurofibrillary Tangles, a later stage lesion present in Alzheimer's disease individuals. Neurofibrillary tangles are composed mainly of abnormally-phosphorylated tau, a neuron-specific phosphoprotein that is the major constituent of neuronal microtubules. In Alzheimer's disease, neurofibrillary tangles are found in the neurons of the cerebral cortex but are most common in the temporal lobe regions, particularly the hippocampus and amygdala. The density and pattern of neurofibrillary tangles correlates with severity of AD. Detection and/or measurement of tau protein and/or phosphorylated tau protein can be accomplished by methods known in the art and are discussed more fully below.

Accordingly, one described method includes a method to delay the development of or reduce the severity of the formation of neurofibrillary tangles in an individual, comprising administering to an individual an amount of at least one omega-3 and/or an omega-6 polyunsaturated fatty acid (PUFA), and/or a precursor or source thereof, to delay the development of or reduce the severity of the formation of neurofibrillary tangles in the individual. In certain examples, the PUFA comprises DHA, DPAn-6, a combination of DPAn-6 and DHA, a combination of DHA and ARA, or a combination of DPAn-6, ARA, and DHA. In one example, the PUFA comprises DHA. In one example, the PUFA comprises DPAn-6. The PUFA is a combination of DHA and DPAn-6.

Another described method includes a method to reduce the level of presenilin-1 (PS1) protein in an individual's neural tissue, comprising administering to an individual an amount of at least one omega-3 and/or an omega-6 polyunsaturated fatty acid (PUFA), and/or a precursor or source thereof, to reduce the level of PS1 protein in the individual. In certain examples, the PUFA comprises DHA, DPAn-6, a combination of DPAn-6 and DHA, a combination of DHA and ARA, or a combination of DPAn-6, ARA, and DHA. In one examples, the PUFA comprises DHA. In one example, the PUFA comprises DPAn-6. In another example, the PUFA is a combination of DHA and DPAn-6.

Presenilin-1 is either considered in the art to be responsible for the gamma-secretase activity that cleaves APP into beta amyloid into pathogenic peptides, or is believed to be an important determinant of the "gamma-secretase" activity necessary for the generation of beta-amyloid. Mutations in the amyloid precursor protein (mAPP) and in presenilin 1 (mPS1) have both been linked to increased production of the beta-amyloid peptide (Aβ). Detection and/or measurement of presenilin-1 expression or activity can be accomplished by methods known in the art that are discussed more fully below.

Also described is a method to delay the onset of and/or reduce the severity of synaptic dysfunction in an individual, comprising administering to the individual an amount of at least one omega-3 and/or an omega-6 polyunsaturated fatty acid (PUFA), and/or a precursor or source thereof, to delay the onset of and/or reduce the severity of synaptic dysfunction in the individual. In this example, the PUFA preferably comprises DHA, DPAn-6, a combination of DPAn-6 and DHA, a combination of DHA and ARA, or a combination of DHA, DPAn-6 and ARA. In preferred embodiments, the PUFA comprises DPAn-6 or a combination of DPAn-6 and DHA. Synaptic dysfunction is a major phenotypic manifestation of Alzheimer's disease neuropathy, and is among the best correlates for the memory and cognitive changes that characterize Alzheimer's disease.

Also described is a method to delay the onset of and/or reduce the severity of dementia, comprising administering to an individual an amount of at least one omega-3 and/or an omega-6 polyunsaturated fatty acid (PUFA), and/or a precursor or source thereof, to delay the onset of and/or reduce the severity of dementia in the individual. In this method, the PUFA preferably comprises DHA, or DPAn-6, a combination of DPAn-6 and DHA, a combination of DHA and ARA, or a combination of DHA, DPAn-6 and ARA. In preferred embodiments, the PUFA comprises DPAn-6 or a combination of DPAn-6 and DHA.

Also described isa method to delay the onset of and/or reduce the severity of a decline in the brain function in an individual comprising administering to the individual an amount of at least one omega-3 and/or an omega-6 polyunsaturated fatty acid (PUFA), and/or a precursor or source thereof to delay the onset of and/or reduce the severity a decline in the brain function in the individual. In this described method, the PUFA preferably comprises DHA or DPAn-6, a combination of DPAn-6 and DHA, a combination of DHA and ARA, or a combination of DHA, DPAn-6 and ARA. In preferred embodiments, the PUFA comprises DPAn-6 or a combination of DPAn-6 and DHA.

Also described is a method to delay or reduce the severity of demyelination in an individual comprising administering to the individual an amount of at least one omega-3 and/or an omega-6 polyunsaturated fatty acid (PUFA), and/or a precursor or source thereof, to delay or reduce the severity of demyelination in the individual. In this method, the PUFA preferably comprises DHA or DPAn-6, a combination of DPAn-6 and DHA, a combination of DHA and ARA, or a combination of DHA, DPAn-6 and ARA. In some methods, the PUFA comprises DPAn-6 or a combination of DPAn-6 and DHA. Myelin is the white matter coating nerve axons and enables efficient neural conduction of impulses between the brain and other parts of the body. Loss of myelin (i.e. demyelination) or abnormal myelin composition may be associated with reduction in neural processing speed or activity and subsequent cognitive decline.

In yet another method, described is a method to treat or prevent a disorder associated with increased amounts of and/or dysfunction of APP, Aβ peptide, PS1 protein, phosphorylated tau protein, and/or tau protein. The described method includes the steps of: (a) identifying individuals with increased amounts of and/or dysfunction of a protein or peptide selected from: APP, Aβ peptide, PS 1 protein, phosphorylated tau protein, tau protein, or combinations thereof; and (b) administering to the individual at least one omega-3 and/or omega-6 polyunsaturated fatty acid (PUFA) and/or a precursor or source thereof. In one described method the PUFA is administered in an amount that is determined be sufficient to reduce the increased amounts or dysfunction of: Aβ peptide, PS1 protein, phosphorylated tau protein, or tau protein. In this described method, the PUFA preferably comprises DHA, DPAn-6, a combination of DPAn-6 and DHA, a combination of DHA and ARA, or a combination of DHA, DPAn-6 and ARA. Preferred PUFAs include DHA, DPAn-6 or a combination of DPAn-6 and DHA..

Also described is a method to treat or prevent a disorder associated with decreased amounts of omega-3 and/or omega-6 polyunsaturated fatty acid (PUFA) and/or a precursor or source thereof. The described method includes the steps of: (a) identifying individuals with decreased amounts of omega-3 and/or omega-6 polyunsaturated fatty acid (PUFA) and/or a precursor or source thereof *(e.g.,* in the tissues or blood); and (b) administering to the individual at least one omega-3 and/or omega-6 polyunsaturated fatty acid (PUFA) and/or a precursor or source thereof in an amount that is determined to be sufficient to compensate for the effects of the decreased amounts of omega-3 and/or omega-6 polyunsaturated fatty acid (PUFA) and/or a precursor or source thereof. In this described method, the PUFA preferably comprises DHA, DPAn-6, a combination of DPAn-6 and DHA, a combination of DHA and ARA, or a combination of DHA, DPAn-6 and ARA. Preferred PUFAs include DPAn-6 or a combination of DPAn-6 and DHA.

Also described isa method to delay or reduce the severity of synaptic dysfunction, decline in brain function, demyelination, and/or dementia or a related disorder in an individual. The described method includes the steps of: (a) identifying individuals with at least one symptom or biological marker indicating a synaptic dysfunction, a decline in brain function, demyelination, and/or dementia or a related disorder; and (b) administering to the individual at least one omega-3 and/or omega-6 polyunsaturated fatty acid (PUFA) and/or a precursor or source thereof in an amount that is determined to be sufficient to delay or reduce the severity of the synaptic dysfunction, decline in brain function, demyelination, and/or dementia or a related disorder. In this method, the PUFA preferably comprises DHA, DPAn-6, a combination of DPAn-6 and DHA, a combination of DHA and ARA, or a combination of DHA, DPAn-6 and ARA. Preferred PUFAs include DHA, DPAn-6 or a combination of DPAn-6 and DHA.

Also described is a method to stabilize and normalize the theta waves on electroencephalogram (EEG) in an individual that has memory loss, early Alzheimer's Disease, or who is a potential sufferer of dementia of any kind, and who has deep, slow, abnormal theta activity on EEG, especially that of the frontal lobes. This described method can also be used to prevent the development of abnormal theta wave activity in those who, by family history or genetic marker, are presumed to be disposed to abnormal theta wave activity. The described method includes the steps of: (a) identifying individuals that have or are predicted to be disposed to develop abnormal theta wave activity; and (b) administering to the individual at least one omega-3 and/or omega-6 polyunsaturated fatty acid (PUFA) and/or a precursor or source thereof in an amount that is determined to be sufficient to stabilize or normalize theta wave activity or to prevent or reduce the development of abnormal theta wave activity in an individual. In this described method, the PUFA preferably comprises DHA, DPAn-6, a combination of DPAn-6 and DHA, a combination of DHA and ARA, or a combination of DHA, DPAn-6 and ARA. Preferred PUFAs include DHA, DPAn-6 or a combination of DPAn-6 and DHA.

In one aspect of the above-described embodiments of the invention, the individual is identified as being susceptible to dementia or pre-dementia. In another aspect, the individual has been positively diagnosed with dementia or pre-dementia. In another aspect, the individual has been positively diagnosed with Alzheimer's disease. Methods to determine whether an individual has or is susceptible to dementia or pre-dementia, including Alzheimer's Disease, include measurement of a biological marker (e.g., APP, Aβ peptide, PS1 protein, phosphorylated tau protein, or tau protein as described herein), or determination of a family history showing dementia, or detection of current Mild Cognitive Impairment, or detection of Age-Related Cognitive Decline.

Mild Cognitive Impairment (MCI) is a form of memory loss that may affect a person's ability to perform on neuropsychological tests. Individuals with MCI usually have impaired memory according to scores on normative standard tests, but do not have impairment in other types of brain function such as planning or attention. These individuals do not have significant problems in managing skills required for everyday living. Individuals with MCI have significantly increased risk of developing Alzheimer's disease or other forms of dementia.

Age-related cognitive decline (ACRD), also referred to as Age-Associated Memory Impairment (AAMI), Age-Consistent Memory Decline, Benign Senescent Forgetfulness (BSF), Cognitive Decline (Age-Related), Forgetfulness (Benign Senescent), or Memory Decline (Age-Consistent)) is not considered to be a disease, although authorities differ on whether age-related cognitive decline is in part related to Alzheimer's disease and other forms of dementia or whether it is a distinct entity. Individuals with ARCD experience deterioration in memory and learning, attention and concentration, thinking, use of language, and other mental functions.

Mild Cognitive impairment (MCI) and Age-Related Cognitive Decline (ARCD) can be identified by various neurologic and cognitive tests including, but not limited to, Mini Mental State Examination (MMSE), Cambridge Neuropsychological Test Automated Battery (CANTAB), Alzheimer's Disease Assessment Scale-cognitive test (ADAScog), the presence of amyloid beta peptides or phosphorylated tau in the cerebral spinal fluid, enlarged brain ventricles determined by Positron Emission Tomography (PET) or Single Photon Emission Computed Tomography (SPECT), detection of the presence of amyloid plaques or neurofibrillary tangles in the brain upon PET or SPECT study, electroencephalogram (EEG), and in some cases, genetic testing. MCI may be determined by a combination of clinical exams, neuropsychological testing that show memory complaints, abnormal memory for age, ability to carry out normal activities of daily living, normal general cognitive function, lack of dementia. In addition, levels of cerebral spinal fluid (CSF) tau and amyloid beta peptides, and brain imaging (PET, SPECT, magnetic resonance imaging (MRI)) may be used to rule out Alzheimer's disease or as secondary risk factors.

Synaptic dysfunction may be measured in a number of ways, including, but not limited to, oxygen and glucose utilization (PET - Positron Emission Tomography, Single Photon Emission Computed Tomography (SPECT)) and functional magnetic resonance spectroscopy (fMRI), electroencephalogram, and long-term potentiation.

Brain function may be measured by a number of methods known in the art, including various cognitive tests to assess speed of information processing, executive function and memory. Examples include, but are not limited to, Mini Mental State Examination (MMSE), Cambridge Neuropsychological Test Automated Battery (CANTAB), Alzheimer's Disease Assessment Scale-cognitive test (ADAScog), Wisconsin Card Sorting Test, Verbal and Figural Fluency Test and Trail Making Test, electroencephalography (EEG), magnetoencephlography (MEG), Positron Emission Tomography (PET), Single Photon Emission Computed Tomography (SPECT), Magnetic Resonance Imaging (MRI), functional Magnetic Resonance Imaging (fMRI), computerized tomography, and long-term potentiation.

EEG, a measure of electrical activity of the brain, is accomplished by placing electrodes on the scalp at various landmarks and recording greatly amplified brain signals.

MEG is allied to EEG in that it measures the magnetic fields that are linked to electrical fields. MEG is used to measure spontaneous brain activity, including synchronous waves in the nervous system (Joliot et al., 1994; Deutsch, 1998).

PET provides a measure of oxygen utilization and glucose metabolism. In this technique, a radioactive positron-emitting tracer is administered, and tracer uptake by the brain is correlated with brain activity. These tracers emit gamma rays which are detected by sensors surrounding the head, resulting in a 3D map of brain activation. As soon as the tracer is taken up by the brain, the detected radioactivity occurs as a function of regional cerebral blood flow (Frackowiak, 1989), and during activation, an increase in CBF and neuronal glucose metabolism can be detected within seconds.

MRI and fMRI capitalize on the fact that one property of atomic nuclei, their spins, can be manipulated by exposing them to a large magnetic force. While the subject lies with his/her head in a powerful magnet (1.5 to 5 Teslas in force), a short-wave radio wave antenna varies the magnetic field in a way that is much weaker than the main magnet. The varying pulse produces a resonance signal from the nuclei that can be quantified in 3D and digitized.

Individuals to be treated using the methods of the invention can also be identified through neuropsychiatric testing, clinical examinations, and individual complaints of loss of cognitive function (e.g., subjective memory loss).

Preferred biological markers to evaluate to identify individuals to be treated or to monitor the treatment of individuals using the present methods include, but are not limited to: APP, Aβ peptide, tau protein, phosphorylated tau protein, PS1, and/or an omega-3 and/or an omega-6 polyunsaturated fatty acid (PUFA) (and preferably DHA, ARA and/or DPAn-6), and/or a precursor or source thereof *(e.g.,* the content of these biomarkers in a biological sample). Preferably, expression (*e*.*g*. RNA or protein detection) or the biological activity of any of these biological markers is measured in a biological sample from the individual prior to the step of administering a PUFA according to the present invention. The amount, expression or biological activity of these biomarkers can be measured after the step of administering a PUFA according to the invention, for example, to monitor the effect of a given method on the treatment of the symptom or condition.

The determination of the significance of the level of amount, expression or biological activity of the biological marker (e.g., determining whether an individual has or is susceptible to dementia or pre-dementia, or determining whether a given protocol of PUFA administration is effective) involves comparing the level of expression and/or a biological activity of a biological marker (also called a biomarker) in the individual sample to a baseline level of amount, expression and/or biological activity of the biomarker in a control or baseline sample. An increase in the amount of the biomarker in the individual sample (for the biomarkers APP, Aβ, APP, Aβ peptide, tau protein, phosphorylated tau protein, and PS 1) as compared to the baseline amount, or a decrease in the amount of an omega-3 and/or an omega-6 polyunsaturated fatty acid (PUFA) as compared to the baseline amount, indicates that the individual is at risk of, or has, synaptic dysfunction, decline in brain function, demyelination, and/or pre-dementia or dementia or a related disorder (e.g., Alzheimer's Disease).

Biomarkers are typically evaluated by evaluating the amount, expression and/or biological activity of the biomarker in a biological sample obtained from the individual. A biological sample can include a cell sample, a tissue sample and/or a bodily fluid sample. The term "cell sample" can be used generally to refer to a sample of any type which contains cells to be evaluated by the present method, including but not limited to, a sample of isolated cells, a tissue sample and/or a bodily fluid sample. As described, a sample of isolated cells is a specimen of cells, typically in suspension or separated from connective tissue which may have connected the cells within a tissue *in vivo,* which have been collected from an organ, tissue or fluid by any suitable method which results in the collection of a suitable number of cells for evaluation by the method of the present invention. The cells in the cell sample are not necessarily of the same type, although purification methods can be used to isolate the cells that are preferably evaluated. Cells can be obtained, for example, by scraping a tissue, processing a tissue sample to release individual cells, or isolating cells from a bodily fluid.

A tissue sample, although similar to a sample of isolated cells, is defined herein as a section of an organ or tissue of the body which typically includes several cell types and/or cytoskeletal structure which holds the cells together. One of skill in the art will appreciate that the term "tissue sample" may be used, in some instances, interchangeably with a "cell sample", although it is preferably used to designate a more complex structure than a cell sample. A tissue sample can be obtained by a biopsy, for example, by cutting, slicing, or using a punch.

A bodily fluid sample, like the tissue sample, also may contain cells and can be obtained by any method suitable for the particular bodily fluid to be sampled. Bodily fluids suitable for sampling include, but are not limited to, blood, cerebrospinal fluid, mucous, seminal fluid, saliva, breast milk, bile and urine. In an example, the biological sample is a blood sample, including any blood fraction (e.g., whole blood, plasma, serum), or a cerebral spinal fluid sample.

In general, the sample type (i.e., cell, tissue or bodily fluid) is selected based on the accessibility of the sample and purpose of the method. Typically, biological samples that can be obtained by the least invasive method are described (e.g., blood), although in some described methods, it may be useful or necessary to obtain a cell or tissue sample for evaluation. Individual tissues may also be evaluated by non-invasive methods, such as imaging methods.

Once a sample is obtained from the individual, the sample is evaluated to detect the presence of, the expression of, or the biological activity of any biomarker described herein, including: APP, Aβ peptide, tau protein, phosphorylated tau protein, and/or PS 1, and/or omega-3 and/or an omega-6 polyunsaturated fatty acid (PUFA), and/or a precursor or source thereof in the sample. Reference to detecting "expression" of a biomarker generally refers to detecting either mRNA transcription or protein translation, including detecting post-translational processing of proteins or peptides (e.g., detecting the amount of protein in a sample). Detecting the "presence" of a biomarker refers to any method of detecting whether a biomarker is present in a sample or not, and can in most cases be used interchangeably with detecting expression or detecting amount. Preferably, the method of detecting the presence, amount, expression or biological activity in the individual is the same or qualitatively equivalent to the method used for detection of presence, amount, expression or biological activity in the sample that is used to establish the baseline or control level of the biomarker.

Methods suitable for detecting biomarker transcription include any suitable method for detecting and/or measuring mRNA levels from a fluid, cell or cell extract. Such methods include, but are not limited to: polymerase chain reaction (PCR), reverse transcriptase PCR (RT-PCR), *in situ* hybridization, Northern blot, sequence analysis, microarray analysis, and detection of a reporter gene. Such methods for detection of transcription levels are well known in the art, and many of such methods are described, for example, in Sambrook et al., Molecular Cloning: A Laboratory Manual, Cold Spring Harbor Labs Press, 1989 and/or in Glick et al., Molecular Biotechnology: Principles and Applications ofRecombinantDNA, ASM Press, 1998; Sambrook et al., *ibid.,* and Glick et al., *ibid.* are incorporated by reference herein in their entireties. Measurement of biomarker transcription is primarily suitable when the sample is a cell or tissue sample; therefore, when the sample is a bodily fluid sample containing cells or cellular extracts, the cells are typically isolated from the bodily fluid to perform the expression assay.

Biomarker expression can also be identified by detection of translation (i.e., detection of protein in the sample). Methods suitable for the detection of protein include any suitable method for detecting and/or measuring proteins from a fluid, cell or cell extract. Such methods include, but are not limited to, Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorption/ionization time-of-flight (MALDI-TOF) mass spectrometry, microcytometry, microarray, microscopy, fluorescence activated cell sorting (FACS), flow cytometry, and protein microchip or microarray, high performance liquid chromatography or size exclusion chromatography. Such methods are well known in the art. Antibodies against the biomarkers described herein have been produced and described in the art and can be used in many of the assays for detection of the biomarkers.

Alternatively, one can readily produce antibodies that selectively bind to the biomarkers using techniques well known in the art. The phrase "selectively binds" refers to the specific binding of one protein to another (e.g., an antibody, fragment thereof, or binding partner to an antigen), wherein the level of binding, as measured by any standard assay (e.g., an immunoassay), is statistically significantly higher than the background control for the assay. For example, when performing an immunoassay, controls typically include a reaction well/tube that contains antibody or antigen binding fragment alone (i.e., in the absence of antigen), wherein an amount of reactivity (e.g., non-specific binding to the well) by the antibody or antigen binding fragment thereof in the absence of the antigen is considered to be background. Binding can be measured using a variety of methods standard in the art including enzyme immunoassays (e.g., ELISA), immunoblot assays, etc.). Antibodies can include polyclonal and monoclonal antibodies, divalent and monovalent antibodies, bi- or multi-specific antibodies, serum containing such antibodies, antibodies that have been purified to varying degrees, and any functional equivalents of whole antibodies (e.g., Fv, Fab, Fab', or F(ab)₂ fragments).

As discussed above, Aβ peptides are found in individuals in one or more different "size forms." These "size forms" can also be detected and compared one to another, or a particular size form of these proteins can be compared to the same moiety in a baseline or control sample. In addition, one can detect the ratio, or profile, of different Aβ peptides or any of the other above-mentioned peptides' size forms in a biological sample from an individual, and compare the profile to that from a baseline control. Particularly useful Aβ size forms (moieties) to detect include the cleavage products of APP having a length of 15 amino acids, 40 amino acids, 42 amino acids or 43 amino acids. Size forms can be detected and distinguished from one another using many of the above-identified methods for detection of protein.

The biomarkers can also be measured in a sample by detecting a biological activity of the biomarker (e.g., a biological activity of a protein). According to the present invention, "biological activity" refers to any biological action of a biomarker described herein, including, but not limited to, enzymatic activity; binding of the protein to another protein *(e.g.,* a receptor, signaling protein, substrate, etc.); activation of a protein; activation of a cell signal transduction pathway; and downstream biological events that occur as a result of the expression, presence, or activation of the biomarker. Methods to detect the biological activity of the biomarkers disclosed herein are known in the art and include, but are not limited to, binding assays, enzyme assays, and phosphorylation assays.

Accordingly, several of the methods of the present invention which include a step of identifying an individual, diagnosing an individual for treatment, and /or monitoring the efficacy of a treatment or protocol, include a step of comparing the level of expression or activity of a biomarker detected in the individual or individual (e.g., APP, Aβ peptide, tau protein, phosphorylated tau protein, PS 1, an omega-3 PUFA and/or an omega-6 PUFA) or the result of a particular test related to synaptic dysfunction, brain function, demyelination, and/or dementia or a related disorder, to a baseline or control level of the expression or activity of the biomarker or test result. According to the present invention, a "baseline level" is a control level, and in some embodiments, a normal level *(e.g.,* a level of the biomarker that is found in or expected to be found in an individual who does not have dementia or pre-dementia or a condition related thereto), of the expression or activity of a biomarker or test result against which a test level of the expression or activity of the biomarker (*i.e.,* in the individual sample) or test result can be compared. Therefore, it can be determined, based on the control or baseline level of the biomarker or test result, whether a sample to be evaluated has a measurable increase, decrease, or substantially no change in the level of expression or activity of the biomarker, or the test result, as compared to the baseline or control level. The term "negative control" used in reference to a baseline level refers to a baseline level established in a sample from an individual or from a population of individuals that is believed to be normal with regard to the expression or activity of the biomarker or with regard to the result of a particular test for function. In another embodiment, a baseline can be indicative of a positive diagnosis of dementia or other condition and/or disease as discussed herein. Such a baseline level, also referred to herein as a "positive control" baseline, refers to a level of a biomarker expression or activity, or a test result, established in a sample from the individual, another individual, or a population of individuals, wherein the level of biomarker expression or activity in the sample or the test result from the control was believed to correspond to a level of biomarker or a test result that indicates a dysfunction, pre-dementia or dementia in the individual. In yet another described method, the baseline level can be established from a previous sample from the individual being tested, so that the biomarker or test result status of an individual can be monitored over time.

The method for establishing a baseline level of the biomarker or test result is selected based on the sample type, the tissue or organ from which the sample is obtained, the status of the individual to be evaluated, and, as discussed above, the focus or goal of the assay (e.g., initial diagnosis, monitoring). Preferably, the described method is the same method that will be used to evaluate the sample or the individual.

In one described method, the baseline level of biomarker amount, expression or biological activity is established in an autologous control sample obtained from the individual. The autologous control sample can be a sample of isolated cells, a tissue sample or a bodily fluid sample, and is preferably a bodily fluid sample *(e.g.,* CSF or blood). According to the described method, and as used in the art, the term "autologous" means that the sample is obtained from the same individual from which the sample to be evaluated is obtained. Preferably, the control sample is obtained from the same fluid, organ or tissue as the sample to be evaluated, such that the control sample serves as the best possible baseline for the sample to be evaluated. This described method is most often used when a previous reading from the individual has been established as either a positive or negative diagnosis for the biomarker. This baseline can then be used to monitor the ongoing progression of the individual toward or away from a disease or condition, or to monitor the success of therapy (e.g., PUFA supplementation). In this described method, a new sample is evaluated periodically (e.g., at annual physicals, which is particularly useful for healthy individuals who have not been diagnosed with dementia or pre-dementia, but wish to monitor for signs of the disease, or on a schedule determined by the clinician treating an individual), and the preventative or therapeutic treatment via fatty acid supplementation is determined at each point. For the first evaluation, an alternate control can be used, as described below, or additional testing may be performed to confirm an initial negative or positive diagnosis with regard to the biomarker or test result and the indication of pre-dementia or dementia, if desired, and this level of biomarker or test result can be used as a baseline thereafter. This type of baseline control is frequently used in other clinical diagnosis procedures where a "normal" level may differ from individual to individual and/or where obtaining an autologous control sample at the time of diagnosis is not possible, not practical or not beneficial.

Another method for establishing a baseline level of biomarker or test result is to establish a baseline level of the biomarker amount, expression or biological activity from control samples or a test result from control subjects, where the control subjects are preferably a population of matched individuals. It is preferred that the control samples are of the same sample type as the sample type to be evaluated for the biomarker amount, expression or biological activity. According to the described method, the phrase "matched individuals" refers to a matching of the control individuals on the basis of one or more characteristics which are suitable for the parameter, type of cell, tissue sample, or bodily fluid sample to be evaluated. For example, control individuals can be matched with the individual to be evaluated on the basis of gender, age, race, or any relevant biological or sociological factor that may affect the baseline of the control individuals and the individual (e.g., preexisting conditions, consumption of particular substances, levels of other biological or physiological factors). For example, levels of the biomarkers described herein in the blood of a normal individual may be higher in individuals of a given classification (e.g., elderly versus teenagers, women versus men). To establish a control or baseline level of biomarker amount, expression or biological activity, samples from a number of matched individuals are obtained and evaluated for biomarker amount, expression or biological activity. The number of matched individuals from whom control samples must be obtained to establish a suitable control level (e.g., a population) can be determined by those of skill in the art, but should be statistically appropriate to establish a suitable baseline for comparison with the individual to be evaluated (i.e., the test individual). The values obtained from the control samples are statistically processed to establish a suitable baseline level using methods standard in the art for establishing such values.

A baseline such as that described above can be a negative control baseline, such as a baseline established from a population of apparently normal control individuals. Alternatively, as discussed above, such a baseline can be established from a population of individuals that have been positively diagnosed as having abnormal levels of biomarkers, dysfunction in a biomarker, synaptic dysfunction, decline in brain function, demyelination, and/or dementia or pre-dementia, so that one or more baseline levels can be established for use in evaluating an individual. The level of biomarker amount, expression or biological activity in the individual sample or the test result from the individual is then compared to each of the baseline levels to determine to which type of baseline (positive or negative) the biomarker level or test result of the individual is statistically closest. It will be appreciated that a given individual sample may fall between baseline levels such that the best diagnosis is that the individual is perhaps beginning to show a dysfunction indicative of the need for at least some fatty acid supplementation, and is perhaps in the process of advancing to the higher stage. The goal of the invention is to reverse, correct, or compensate for such advancing disease.

It will be appreciated by those of skill in the art that a baseline need not be established for each assay or evaluation as the assay or evaluation is performed but rather, a baseline can be established by referring to a form of stored information regarding a previously determined baseline level for a given control sample, such as a baseline level established by any of the above-described methods. Such a form of stored information can include, for example, but is not limited to, a reference chart, listing or electronic file of population or individual data regarding "normal" (negative control) or positive controls; a medical chart for the individual recording data from previous evaluations; or any other source of data regarding baseline biomarker expression or activity or a test result that is useful for the individual to be diagnosed or evaluated.

In comparing the results from the individual test or individual sample to the baseline control(s), it is determined whether the test sample has a measurable decrease or increase in biomarker amount, expression or biological activity over the baseline level, or whether there is no statistically significant difference between the test and baseline levels. After this step, the final step of making a diagnosis, treating an individual, monitoring the individual, or determining further treatment of the individual can be performed.

Detection of an increased level of APP, Aβ peptide, tau protein, phosphorylated tau protein, and/or PS1 amount, expression or biological activity, or detection of increased processing of APP into the more toxic size forms of Aβ, in the sample to be evaluated (i.e., the test sample), or detection of an increase or no decline in synaptic dysfunction, decline in brain function, demyelination, and/or a symptom of dementia, or detection of reduced amounts of omega-3 or omega-6 PUFAs, as compared to the baseline level, generally indicates that, as compared to the baseline sample or baseline result, the individual may have increased susceptibility to or have any of the diseases or conditions discussed herein (e.g. dementia). If the baseline sample is a previous sample or evaluation from the individual (or a population control) and is representative of a positive diagnosis of dementia or pre-dementia in the individual, a detection of increased biomarker amount, expression or biological activity in the sample or detection of an increase or no decline in synaptic dysfunction, decline in brain function, demyelination, and/or dementia, or detection of decreased omega-3 or omega-6 PUFAs, as compared to the baseline indicates that the individual condition is worsening, rather than improving and that treatment should be reevaluated or adjusted.

Detection of a normal or healthy amount of APP, Aβ peptide, tau protein, phosphorylated tau protein, and/or PS 1 amount, expression or biological activity, detection of the processing of APP to the less toxic size form of Aβ in the sample to be evaluated (i.e., the test sample), or detection of: reduced or no synaptic dysfunction, reduced or no decline in brain function, reduced demyelination, and/or reduced or no symptoms of dementia, or detection of normal or increased levels of or detection of omega-3 or omega-6 PUFAs, as compared to the baseline level indicates that, as compared to the baseline sample, the individual may have decreased susceptibility to or does not have any of the diseases or conditions discussed herein. If the baseline sample is a previous sample from the individual (or from a population control) and is representative of a positive diagnosis of dementia or pre-dementia in the individual (i.e., a positive control), a detection of this result in the sample or individual as compared to the baseline indicates that the individual is experiencing an improvement in the diseases or conditions discussed herein.

Finally, detection of a biomarker expression or activity, or a test result, that is not statistically significantly different than the biomarker amount, expression or biological activity or the test result in the baseline sample indicates that, as compared to the baseline sample, no difference in the diseases or conditions discussed herein is indicated in the individual. If the baseline sample is a previous sample from the individual (or from a population control) and is representative of a positive diagnosis of dementia or pre-dementia in the individual (i.e., a positive control), a detection of biomarker expression or activity or a test result that is not statistically significantly different than the baseline indicates that the individual has no change in the condition, which might suggest to a clinician that a treatment currently being prescribed, for example, is ineffective in controlling the condition.

In order to establish a diagnosis of a change as compared to a baseline level of expression or activity of a biomarker or with regard to a test result, the level of biomarker expression or activity or the test result value is changed as compared to the established baseline by an amount that is statistically significant (i.e., with at least a 95% confidence level, or p<0.05). Preferably, detection of at least about a 5% change, and more preferably, at least about a 10% change, and more preferably, at least about a 20% change, and more preferably, at least about a 30% change, and more preferably, at least about a 40% change, and more preferably, at least about a 50% change, in biomarker amount, expression or biological activity in the sample or in a test result value, as compared to the baseline level, results in a diagnosis of a difference between the test sample and the baseline sample. In one described example, a 1.5 fold change in biomarker amount, expression or biological activity in the sample or in a test result value as compared to the baseline level, and more preferably, detection of at least about a 3 fold change, and more preferably at least about a 6 fold change, and even more preferably, at least about a 12 fold change, and even more preferably, at least about a 24 fold change, results in a diagnosis of a significant change in biomarker expression or activity or in a test result value, as compared to the baseline sample.

The described methods include monitoring the efficacy of the administration of PUFAs on the levels of APP, Aβ peptide, tau protein, phosphorylated tau protein, PS1 protein, and in some embodiments, on omega-3 PUFA and/or omega-6 PUFA levels, in the individual at least one time subsequent to the step of administering. Efficacy can be measured by a change in the amount and/or biological activity of one or more biomarkers, improvement of mild cognitive impairment, improvement of age-related cognitive decline, and/or any other methods known in the art and as discussed in more detail above. The method may optionally further include adjusting the administration of the PUFA to the individual in subsequent treatments based on the results of the monitoring of efficacy of the treatment.

The described diagnostic and monitoring methods have several different uses. First, the method can be used to diagnose and monitor a subset of individuals who have excess APP, Aβ peptide, tau protein, phosphorylated tau protein, and/or PS 1 expression or dysfunction within a larger pool of individuals having a given condition (e.g., a neurological condition), in order to identify those individuals who are most likely to be benefited by the methods of the present invention. Indeed, the described method is believed to be effective early in the development of dementia and pre-dementia, before overt symptoms of cognitive decline may be apparent. The method can also be used to diagnose and monitor individuals by identifying individuals that have PUFA deficiency (*e.g*., deficiency in DHA, DPAn-6 and/or ARA), or the potential for PUFA deficiency, in an individual. The individual can be an individual who is suspected of having a PUFA deficiency or an individual who is presumed to be healthy, but who is undergoing a routine screening for PUFA deficiency. The individual can also be an individual who has previously been diagnosed with PUFA deficiency and treated, and who is now under routine surveillance for recurring PUFA deficiency.

The terms "diagnose," "diagnosis," "diagnosing" and variants thereof refer to the identification of a disease or condition on the basis of its signs and symptoms. As used herein, a "positive diagnosis" indicates that the disease or condition, or a potential for developing the disease or condition, has been identified. In contrast, a "negative diagnosis" indicates that the disease or condition, or a potential for developing the disease or condition, has not been identified. In the case of a positive diagnosis, an individual can be prescribed treatment to reverse or eliminate the signs of dementia or pre-dementia, the PUFA deficiency, and/or the abnormal amount, expression and/or activity of APP, Aβ peptide, tau protein, phosphorylated tau protein, and/or PS1. In the case of a negative diagnosis (i.e., a negative assessment), the individual is typically not prescribed any treatment, or may be placed on low level PUFA supplementation, but may be reevaluated at one or more time points in the future to again assess the level of biomarkers or indicators of synaptic dysfunction, brain function, demyelination, and/or a symptom of dementia. Baseline levels for this particular described example of the method of assessment are typically based on a "normal" or "healthy" sample from the same bodily source as the test sample (i.e., the same tissue, cells or bodily fluid), as discussed in detail below.

The described method can also be used to is used to monitor the success, or lack thereof, of a treatment for dementia or pre-dementia or a symptom or indicator thereof (*e.g.,* a biomarker level as described herein) in an individual that has been given a negative diagnosis with regard to the conditions described herein. This allows the physician or care provider to monitor the success, or lack of success, of a treatment (e.g., PUFA supplementation) that the individual is receiving for a given condition and can help the physician to determine whether the treatment should be modified (e.g., whether PUFA supplementation should be increased, decreased, or remain substantially the same). In one example, the method includes additional steps of providing other treatment to the individual that is useful for the treatment and/or prevention of dementia or pre-dementia or symptoms thereof.

Administration of PUFA(s) in order to regulate the level of a biomarker or PUFA tissue level, or to delay the onset/development of or reduce the severity of a dementia or pre-dementia or a symptom or condition related thereto should provide some benefit (e.g., therapeutic or health benefit) to both healthy, normal individuals, as well as individuals who may be developing dementia or a pre-dementia, or who have dementia or pre-dementia. As such, a therapeutic benefit is not necessarily a cure for a particular disease or condition, but rather, preferably encompasses a result which most typically includes alleviation of the disease or condition, elimination of the disease or condition, reduction of a symptom associated with the disease or condition, delay of onset or development of a symptom of a disease, condition or symptom, prevention or alleviation of a secondary disease or condition resulting from the occurrence of a primary disease or condition, and/or prevention of the disease or condition. As used herein, the phrase "protected from a disease" refers to reducing the symptoms of the disease or condition, reducing the occurrence of the disease or condition, delaying the onset or development of the disease or conditions, and/or reducing the severity of the disease or condition. As such, to protect an individual from a disease includes both preventing or delaying or reducing disease occurrence (prophylactic treatment) and treating an individual that has a disease (therapeutic treatment). A beneficial effect can easily be assessed by one of ordinary skill in the art and/or by a trained clinician who is treating an individual. The term, "disease" refers to any deviation from the normal health of a mammal and includes a state when disease symptoms are present, as well as conditions in which a deviation has occurred, but symptoms are not yet manifested.

Polyunsaturated fatty acids (PUFAs) are critical components of membrane lipids in most eukaryotes (Lauritzen et al., Prog. Lipid Res. 40 1 (2001); McConn et al., Plant J. 15, 521 (1998)) and are precursors of certain hormones and signaling molecules (Heller et al., Drugs 55, 487 (1998); Creelman et al., Annu. Rev. Plant Physiol. Plant Mol. Biol. 48, 355 (1997)).

PUFAs are fatty acids with a carbon chain length of at least 16 carbons, and more preferably at least 18 carbons, and more preferably at least 20 carbons, and more preferably 22 or more carbons, with at least 3 or more double bonds, and preferably 4 or more, and more preferably 5 or more, and even more preferably 6 or more double bonds, wherein all double bonds are in the *cis* configuration. Reference to long chain polyunsaturated fatty acids (LCPUFAs) herein more particularly refers to fatty acids of 18 and more carbon chain length, and preferably 20 and more carbon chain length, containing 3 or more double bonds. LCPUFAs of the omega-6 series include: gamma-linolenic acid (C18:3), di-homo-gammalinolenic acid (C20:3n-6), arachidonic acid (C20:4n-6), adrenic acid (also called docosatetraenoic acid or DTA) (C22:4n-6), and docosapentaenoic acid (C22:5n-6). The LCPUFAs of the omega-3 series include: alpha-linolenic acid (C18:3), eicosatrienoic acid (C20:3n-3), eicosatetraenoic acid (C20:4n-3), eicosapentaenoic acid (C20:5n-3), docosapentaenoic acid (C22:5n-3), and docosahexaenoic acid (C22:6n-3). The LCPUFAs also include fatty acids with greater than 22 carbons and 4 or more double bonds including but not limited to C28:8(n-3).

As used herein, the term "lipid" includes "lipid" includes phospholipids (PL); free fatty acids; esters of fatty acids; triacylglycerols (TAG); diacylglycerides; monoacylglycerides; phosphatides; waxes (esters of alcohols and fatty acids); sterols and sterol esters; carotenoids; xanthophylls (e.g., oxycarotenoids); hydrocarbons; and other lipids known to one of ordinary skill in the art. The terms "polyunsaturated fatty acid" and "PUFA" include not only the free fatty acid form, but other forms as well, such as the TAG form and the PL form.

Blends of fatty acids and particularly, omega-3 fatty acids and omega-6 fatty acids can be used in the methods of the invention. Described PUFAs include omega-3 and omega-6 polyunsaturated fatty acids with three or more double bonds. Omega-3 PUFAs are polyethylenic fatty acids in which the ultimate ethylenic bond is three carbons from and including the terminal methyl group of the fatty acid and include, for example, docosahexaenoic acid C22:6(n-3) (DHA) and omega-3 docosapentaenoic acid C22:5(n-3) (DPA n-3). Omega-6 PUFAs are polyethylenic fatty acids in which the ultimate ethylenic bond is six carbons from and including the terminal methyl group of the fatty acid and include, for example, arachidonic acid C20:4(n-6) (ARA); C22:4(n-6), omega-6 docosapentaenoic acid C22:5(n-6) (DPAn-6); and dihomogammalinolenic acid C20:3(n-6)(dihomo GLA). The present invention uses a composition comprising docosahexaenoic acid (DHA) and docosapentaenoic acid n-6 (DPAn-6), wherein the ratio of DHA to DPAn-6 is from about 1:1 to about 10:1

Any source of PUFA can be used in the compositions and methods of the present invention, including, for example, animal, plant and microbial sources. Preferred polyunsaturated fatty acid (PUFA) sources can be any sources of PUFAs that are suitable for use in the present invention. Preferred polyunsaturated fatty acids sources include biomass sources, such as animal, plant and/or microbial sources.

Examples of animal sources include aquatic animals (e.g., fish, marine mammals, crustaceans, rotifers, etc.) and lipids extracted from animal tissues (e.g., brain, liver, eyes, etc.). Examples of plant sources include macroalgae, flaxseeds, rapeseeds, corn, evening primrose, soy and borage. Examples of microorganisms include microalgae, protists, bacteria and fungi (including yeast). The use of a microorganism source, such as microalgae, can provide organoleptic advantages, i.e., fatty acids from a microorganism source may not have the fishy taste and smell that fatty acids from a fish source tend to have. More preferably, the long-chain fatty acid source comprises microalgae or microalgal oils.

Preferably, when microorganisms are the source of long-chain fatty acids, the microorganisms are cultured in a fermentation medium in a fermentor. Alternatively, the microorganisms can be cultured photosynthetically in a photobioreactor or pond. Preferably, the microorganisms are lipid-rich microorganisms, more preferably, the microorganisms are selected from the group consisting of microalgae, bacteria, fungi and protists, more preferably, the microorganisms are selected from the group consisting of golden algae, green algae, dinoflagellates, yeast, fungi of the genus *Mortierella* and Stramenopiles. Preferably, the microorganisms comprise microorganisms of the genus Crypthecodinium and order Thraustochytriales and filamentous fungi of the genus Mortierella, and more preferably, microorganisms are selected from the genus *Thraustochytrium*, *Schizochytrium*, *Ulkenia* or mixtures thereof, more preferably, the microorganisms are selected from the group consisting of microorganisms having the identifying characteristics ofATCC number 20888, ATCC number 20889, ATCC number 20890, ATCC number 20891 and ATCC number 20892, strains of *Mortierella schmuckeri* and *Mortierella alpina,* strains of *Crypthecodinium cohnii,* mutant strains derived from any of the foregoing, and mixtures thereof. Information regarding such algae can be found in U.S. Patent Nos. 5,407,957, 5,130,242 and 5,340,594, which are incorporated herein by reference in their entirety.

According to the invention, the term "marine microalgae" include microalgae that can naturally inhabit marine or saline environments. Marine microalgae, as referenced herein, include microorganisms of the order Thraustochytriales (also referred to herein as Thraustochytrids) and microorganisms of the order Labyrinthulales (also referred to herein as Labyrinthulids). It is recognized that at the time of this invention, revision in the taxonomy of Thraustochytrids places the genus *Labyrinthuloides* in the family of Labyrinthulaceae and confirms the placement of the two families Thraustochytriaceae and Labyrinthulaceae within the Stramenopile lineage. It is noted that the Labyrinthulaceae are sometimes commonly called labyrinthulids or labyrinthula, or labyrinthuloides and the Thraustochytriaceae are commonly called thraustochytrids. The members of the family Labyrinthulaceae have previously been considered to be members of the order Thraustochytriales, but the family is now considered to be a member of the order Labyrinthulales, and both Labyrinthulales and Thraustochytriales are considered to be members of the phylum Labyrinthulomycota. Accordingly, as used herein, the term "Thraustochytrid" refers to any members of the order Thraustochytriales, which includes the family Thraustochytriaceae, and the term "Labyrinthulid" refers to any member of the order Labyrinthulales, which includes the family Labyrinthulaceae.

Developments have resulted in frequent revision of the taxonomy of the Thraustochytrids (thraustochytrids). Taxonomic theorists generally place Thraustochytrids with the algae or algae-like protists. However, because of taxonomic uncertainty, it would be best for the purposes of the present invention to consider the strains described in the present invention as Thraustochytrids to include the following organisms: Order: Thraustochytriales; Family: Thraustochytriaceae; Genera: *Thraustochytrium* (Species: sp., *arardimentale, aureum*, *benthicola, globosum*, *kinnei, motivum*, *multirudimentale*, *pachydermum*, *proliferum*, *roseum*, *striatum*), *Ulkenia* (previously considered by some to be a member of *Thraustochytrium*) (Species: sp., *amoeboidea, kerguelensis*, *minuta, profunda, radiata, sailens, sarkariana, schizochytrops*, *visurgensis*, *yorkensis), Schizochytrium* (Species: sp., *aggregatum*, *limnaceum*, *mangrovei, minutum*, *octosporum*), *Japonochytrium* (Species: sp., *marinum*), *Aplanochytrium* (Species: sp., *haliotidis, kerguelensis*, *profunda, stocchinoi), Althornia* (Species: sp., *crouchii*), or *Elina* (Species: sp., *marisalba, sinorifica).*

Strains described in the present invention as Labyrinthulids include the following organisms: Order: Labyrinthulales, Family:Labyrinthulaceae, Genera: *Labyrinthula* (Species: sp., *algeriensis, coenocystis, chattonii, macrocystis, macrocystis atlantica, macrocystis macrocystis, marina, minuta, roscoffensis, valkanovii, vitellina, vitellina pacifica, vitellina vitellina, zopfii*), *Labyrinthuloides* (Species: sp., *haliotidis, yorkensis), Labyrinthomyxa* (Species: sp., *marina), Diplophrys* (Species: sp., *archeri*)*, Pyrrhosorus* (Species: sp., *marinus*)*, Sorodiplophrys* (Species: sp., *stercorea)* or *Chlamydomyxa* (Species: sp., *labyrinthuloides, montana)* (although there is currently not a consensus on the exact taxonomic placement of *Pyrrhosorus*, *Sorodiplophrys* or *Chlamydomyxa*).

The present invention relates to the use of docosahexaenoic acid (DHA; at least about 10, about 20, about 30 or about 35 weight percent) and docosapentaenoic acid (DPAn-6; at least about 5, about 10, about 15, or about 20 weight percent). Also described is arachidonic acid (ARA; at least about 20, about 30, about 40 or about 50 weight percent). Other PUFAs may include eicosapentaenoic acid (EPA). PUFAs include free fatty acids and compounds comprising PUFA residues, including phospholipids; esters of fatty acids; triacylglycerols; diacylglycerides; monoacylglycerides; lysophospholipids; phosphatides; etc.

Sources of phospholipids include poultry eggs, enriched poultry eggs, algae, fish, fish eggs, and genetically engineered (GE) plant seeds or microalgae. Particularly preferred sources of PUFAs, including DHA include, but are not limited to, fish oil, marine microalgae, and plant oils, including oils from genetically engineered microoalgae and plants. Preferred precursors of the PUFA, DHA, include, but are not limited to, α-linolenic acid (LNA); eicosapentaenoic acid (EPA); docosapentaenoic acid (DPA); blends of LNA, EPA, and/or DPA.

In accordance with the present invention, the long-chain fatty acids that are used in the supplements and therapeutic compositions described herein are in a variety of forms. For example, such forms include, but are not limited to: a highly purified algal oil comprising the PUFA, a plant oil comprising the PUFA, triglyceride oil comprising the PUFA, phospholipids comprising the PUFA, a combination of protein and phospholipids comprising the PUFA, dried marine microalgae comprising the PUFA, sphingolipids comprising the PUFA, esters of the PUFA, free fatty acid, a conjugate of the PUFA with another bioactive molecule, and combinations thereof. Long chain fatty acids can be provided in amounts and/or ratios that are different from the amounts or ratios that occur in the natural source of the fatty acids, such as by blending, purification, enrichment and genetic engineering of the source. Bioactive molecules can include any suitable molecule, including, but not limited to, a protein, an amino acid (e.g. naturally occurring amino acids such as DHA-glycine, DHA-lysine, or amino acid analogs), a drug, and a carbohydrate. The forms outlined herein allow flexibility in the formulation of foods with high sensory quality, dietary supplements, and pharmaceutical agents. For example, currently available microalgal oils contain about 40% DHA. These oils can be turned into ester form and then purified using techniques such as molecular distillation to extend the DHA content to 70% and greater, providing a concentrated product that can be useful in products with size constraints, i.e. small serving sizes such as infant foods or dietary supplements with limited feasible pill size. Use of oil and phospholipid combinations helps to enhance the oxidative stability and therefore sensory and nutritional quality of microalgal oil. Oxidative breakdown compromises the nutritional and sensory quality of PUFAs in triglyceride form. By employing the phospholipid form, the desired PUFAs are more stable and the fatty acids are more bioavailable then when in the triglyceride form. Although microbial oils are more stable than typical fish oils, both are subject to oxidative degradation. Oxidative degradation decreases the nutritional value of these fatty acids. Additionally, oxidized fatty acids are believed to be detrimental to good health. The use of phospholipid DHA/DPA(n-6), a more stable fatty acid system, enhances the health and nutritional value of these supplements. Phospholipids are also easier to blend into aqueous systems than are triglyceride oils. Use of protein and phospholipid combinations allows for the formulation of more nutritionally complex foods as both protein and fatty acids are provided. Use of dried marine microalgae provides high temperature stability for the oil within it and is advantageous for the formulation of foods baked at high temperature.

In one embodiment of the invention, a source of the desired phospholipids includes purified phospholipids from eggs, plant oils, and animal organs prepared via the Friolex process and phospholipid extraction process (PEP) (or related processes) for the preparation of nutritional supplements rich in DHA, DPA, ARA and/or dihomo-GLA. The Friolex and PEP, and related processes are described in greater detail in PCT Patent Nos. PCT/IB01/00841, entitled "Method for the Fractionation of Oil and Polar Lipid-Containing Native Raw Materials", filed April 12, 2001, published as WO 01/76715 on October 18, 2001; PCT/IB01/00963, entitled "Method for the Fractionation of Oil and Polar Lipid-Containing Native Raw Materials Using Alcohol and Centrifugation", filed April 12, 2001, published as WO 01/76385 on October 18, 2001; and PCT/DE95/01065 entitled "Process For Extracting Native Products Which Are Not Water-Soluble From Native Substance Mixtures By Centrifugal Force", filed August 12, 1995, published as WO 96/05278 on February 22, 1996.

Preferably, the highly purified algal oil comprising the desired PUFA in triglyceride form, triglyceride oil combined with phospholipid, phospholipid alone, protein and phospholipid combination, or dried marine microalgae, comprise DHA and DPAn-6. More preferably, the highly purified algal oil comprising the desired PUFA in triglyceride form, triglyceride oil combined with phospholipid, phospholipid alone, protein and phospholipid combination, or dried marine microalgae, comprise DHA and DPAn-6. More preferably, the highly purified algal oil comprising the desired PUFA in triglyceride form, triglyceride oil combined with phospholipid, phospholipid alone, protein and phospholipid combination, or dried marine microalgae, comprise DHA and DPAn-6.

The PUFA comprises a combination of DPAn-6 and DHA. The inventor has found that this combination of PUFAs delivers a surprising, unexpected benefit over administration of DHA alone. It is unexpected that the combination of DPAn-6 and DHA in particular would deliver any benefits over administration of DHA alone or DHA in combination with other omega-6 PUFAs. It is surprising because the roles of DPA n-6 in tissues rise when insufficient dietary DHA is consumed. The rise in DPAn-6 in tissues of a DHA-deficient animal is often associated with sub-optimal tissue function. In the present examples, animals that were provided with sufficient dietary preformed DHA in combination with DPAn-6 had significantly increased DPAn-6 levels in tissue and superior ARA levels compared to animals fed DHA alone. Animals with higher blood DHA and ARA levels tended to have greater reduction in PS-1, the protein associated with cleavage of APP into toxic amyloid peptides. Furthermore, animals that were provided with sufficient dietary preformed DHA in combination with DPAn-6 had reduced phosphorylated tau levels, which is associated with the development of neurofibrillary tangles in patients with dementia.

In another preferred embodiment, the PUFA comprises an oil or formulation comprising about 30% or more, about 35% or more, about 40% or more, about 45% or more, about 50% or more, about 55% or more, about 60% or more, about 65% or more, about 70% or more, about 75% or more, or about 80% or more of a combination of DPAn-6 and DHA. The ratio of DHA to DPAn-6 in the oil or formulation is between about 1:1 to about 10:1, or any ratio between 1:1 and 10:1.

The PUFA may comprise a combination of DHA, ARA and DPAn-6, which will deliver a surprising, unexpected benefit over administration of DHA alone, for the reasons discussed above. In another preferred embodiment, the PUFA comprises an oil or formulation comprising about 30% or more, about 35% or more, about 40% or more, about 45% or more, about 50% or more, about 55% or more, about 60% or more, about 65% or more, about 70% or more, about 75% or more, or about 80% or more of a combination of DPAn-6, ARA and DHA. Preferably, the ratio of DHA to ARA to DPAn-6 is about 1:1:1 to about 10:1:1 or any ratio in between 1:1:1 and 10:1:1.

Daily PUFA intake preferably ranges from about 0.025 mg to about 15 grams daily, including any increment in between, in 0.005 increments (e.g., 0.025,0.030,0.035, etc.). In one embodiment, a PUFA is administered in a dosage of from about 0.05 mg of the PUFA per kg body weight of the individual to about 200mg of the PUFA per kg body weight of the individual or higher, including any increment in between, in 0.01 mg increments (e.g., 0.06 mg, 0.07 mg, etc.), or in amounts ranging between about 50 mg and about 20,000 mg per subject per day *(e.g.,* by oral, injection, emulsion or total parenteral nutrition, topical, intraperitoneal, placental, transdermal, or intracranial delivery). In another embodiment, a PUFA is administered in a dosage of from about 0.45 mg PUFA per kg body weight per day to about 275 mg PUFA per kg body weight per day. In another embodiment, a PUFA is administered in a dosage of from about 0.025 mg PUFA per kg body weight per day to about 275 mg PUFA per kg body weight per day, including any increment in between, in 0.005 increments (e.g., 0.025, 0.030, 0.035, etc.). In another embodiment, a PUFA is administered in a dosage of from about 0.05 mg PUFA per kg body weight per day to about 275 mg PUFA per kg body weight per day. A typical capsule DHA supplement for example, can be produced in 100mg to 200mg doses per capsule, although the invention is not limited to capsule forms or capsules containing these amounts of DHA or another PUFA.

Although fatty acids such as DHA can be administered topically or as an injectable, the most preferred route of administration is oral administration. Preferably, the fatty acids (e.g., PUFAs) are administered to individuals in the form of nutritional supplements and/or foods and/or pharmaceutical formulations and/or beverages, more preferably foods, beverages, and/or nutritional supplements, more preferably, foods and beverages, more preferably foods. A preferred type of food is a medical food (e.g., a food which is in a formulation to be consumed or administered externally under the supervision of a physician and which is intended for the specific dietary management of a disease or condition for which distinctive nutritional requirements, based on recognized scientific principles, are established by medical evaluation.) For infants, the fatty acids are administered to infants as infant formula, weaning foods, jarred baby foods, and infant cereals.

Any biologically acceptable dosage forms, and combinations thereof, are contemplated by the inventive subject matter. Examples of such dosage forms include, without limitation, chewable tablets, quick dissolve tablets, effervescent tablets, reconstitutable powders, elixirs, liquids, solutions, suspensions, emulsions, tablets, multi-layer tablets, bi-layer tablets, capsules, soft gelatin capsules, hard gelatin capsules, caplets, lozenges, chewable lozenges, beads, powders, granules, particles, microparticles, dispersible granules, cachets, douches, suppositories, creams, topicals, inhalants, aerosol inhalants, patches, particle inhalants, implants, depot implants, ingestibles, injectables, infusions, health bars, confections, cereals, cereal coatings, foods, nutritive foods, functional foods and combinations thereof. The preparations of the above dosage forms are well known to persons of ordinary skill in the art. Preferably, a food that is enriched with the desired PUFA is selected from the group including, but not limited to: baked goods and mixes; chewing gum; breakfast cereals; cheese products; nuts and nut-based products; gelatins, pudding, and fillings; frozen dairy products; milk products; dairy product analogs; soft candy; soups and soup mixes; snack foods; processed fruit juice; processed vegetable juice; fats and oils; fish products; plant protein products; poultry products; and meat products.

Another embodiment of the present invention includes a pharmaceutical composition comprising docosahexaenoic acid (DHA) and docosapentaenoic acid n-6 (DPAn-6) in a ratio of from about 1:1 to about 10:1 in an oral dosage for the treatment of dementia or pre-dementia in a human.

Therapeutic compounds appropriate to use with the present invention include any therapeutic which can be used to protect an individual against any of the conditions or diseases discussed herein, and may include a protein, an amino acid, a drug, other natural products and a carbohydrate. Such therapeutic compounds will be well known to those of skill in the art for the particular disease or condition being treated. Some preferred therapeutic compounds to include a composition or formulation of the invention include, but are not limited to: Tacrine (COGNEX); Donepezil (ARICEPT); Rivastigmine (EXELON); Galantamine (REMINYL); Memantine (AKATINOL); Neotropin; Nootropics; Alpha-tocopherol (vitamin E); Selegeline (ELDEPRYL); non-steroidal anti-inflammatory agents (NSAIDS); Gingko biloba; estrogen; β-secretase inhibitors; vaccines, including lipid or liposome-based vaccines, that dissolve plaques in the brain; B complex vitamins; calcium channel blockers; HMG CoA reductase inhibitors; statins; policosanols; fibrates; Clioquinol; and other natural products (e.g., curcumin, lignans, phytoestrogens, phytosterols; niacin, and vitamin supplements).

Dosages and routes of administration are known in the art and may be determined by those of skill in the art.

The present invention also includes a method of making any of the above-described compositions of the invention, such as by combining the components of the composition into any suitable delivery form using any suitable method known in the art.

According to the present invention, the methods of the present invention are suitable for use in an individual that is a member of the Vertebrate class, Mammalia, including, without limitation, primates, livestock and domestic pets (e.g., a companion animal). Most typically, an individual will be a human individual. The term "individual" can be interchanged with the term "subject" or "patient" and refers to the subject of a protocol or method according to the invention. Accordingly, an individual can include a healthy, normal (non-diseased) individual, as well as an individual who has or is at risk of developing pre-dementia or dementia or a symptom or indicator thereof as described herein.

The following examples are provided for the purpose of illustration and are not intended to limit the scope of the present invention.

### Examples

The following Materials and Methods were used in the Examples described below.

### Generation of 3XTg-AD Mice

Generation of the 3XTg-AD mice was as described in Oddo, "Triple-transgenic Model of Alzheimer's Disease with Plaques and Tangles: Intracellular Aβ and Synaptic Dysfunction," Neuron, Vol. 39, 409-21 (2003). Briefly, human APP (695 isoform) cDNA harboring the Swedish double mutation (KM670/671NL) was subcloned into exon 3 of the Thy1.2 expression cassette. Human four-repeat tau without amino terminal inserts (4R0N) harboring the P30IL mutation was also subcloned into Thy1.2 expression cassette. After restriction digestion to liberate the transgene, each fragment was purified by sucrose gradient fractionation, followed by overnight dialysis in injection buffer (10 mM Tris [pH 7.5], 0.25 mM EDTA). Equal molar amounts of each construct were co-microinjected into the pronuclei of single cell embryos harvested from homozygous PS 1M146V knockin mice (Guo et al., Arch. Pathol. Lab. Med. 125, 489-492(2001). The PS1 knockin mice were originally generated as a hybrid 129/C57BL6 background. Transgenic mice were identified by Southern blot analysis of tail DNA as described previously (Sugarman et al. Natl. Acad. Sci 99, 6334-6339 (2002). Founder mice were backcrossed to the parental PS1 knockin mice.

### Diets

All mice described in these examples received experimental diets starting at 3 months of age to completion of the experiment at 6, 9, or 12 months of age. The diet formulation was the AIN-76 rodent diet containing 5% total fat. The target fatty acid composition of each diet is described in TABLE 1 and was achieved by blending a combination of vegetable and microalgal oils as outlined in TABLE 2. Because the study was initially performed in a blinded manner, the diets were color coded as follows, which is referenced in some tables and figures: BLUE (comparative) diet (Corn/Soy); YELLOW comparative diet (DHASCO^{®}; also known as DHA-supplemented diet, or DHA-enriched diet); GREEN diet (DHA™-S; also known as DHA- and DPAn-6-supplemented diet or DHA- and DPAn-6-enriched diet); and RED comparative diet (DHASCO^{®}/ARASCO^{®}; also known as DHA- and ARA-supplemented diet or DHA- and ARA-enriched diet). Collectively, the diets containing DHASCO^{®}, DHA™-S, or DHASCO^{®}/ARASCO^{®} can be referred to as diets containing microalgal oils.

DHASCO^{®} is an oil derived from *Crypthecodinium cohnii* containing high amounts of docosahexaenoic acid (DHA), and more specifically contains the following approximate exemplary amounts of these fatty acids, as a percentage of the total fatty acids: Myristic acid (14:0) 10-20%; Palmitic acid (16:0) 10-20%; Palmitoleic acid (16:1) 0-2%; Stearic acid (18:0) 0-2%; Oleic acid (18:1) 10-30%; Linoleic acid (18:2) 0-5%; Arachidic acid (20:0) 0-1%; Behenic acid (22:0) 0-1%; Docosapentaenoic acid (22:5) 0-1%; **Docosahexanoic acid (22:6) (DHA) 40-45%;** Nervonic acid (24:1) 0-2%; and Others 0-3%.

DHA™-S (also formerly referenced as DHASCO^{®}-S) is an oil derived from the Thraustochytrid, *Schizochytrium* sp., that contains a high amount of DHA and also contains docosapentaenoic acid (n-6) (DPAn-6), and more specifically contains the following approximate exemplary amounts of these fatty acids, as a percentage of total fatty acids: Myristic acid (14:0) 8.71%; Palmitic acid (16:0) 22.15%; Stearic acid (18:0) 0.66%; Linoleic acid (18:2) 0.46%; Arachidonic acid (20:4) 0.52%; Eicosapentenoic acid (20:5, n-3) 1.36%; **Docosapentaenoic acid (22:5, n-6) (DPAn-6) 16.28%; Docosahexaenoic acid (DHA) (22:6, n-3) 41.14%;** and Others 8%.

ARASCO^{®} is an oil derived from *Mortierella alpina* that contains a high amount of Arachidonic Acid (ARA), and more specifically contains the following approximate exemplary amounts of these fatty acids: Myristic acid (14:0) 0-2%; Palmitic acid (16:0) 3-15%; Palmitoleic acid (16:1) 0-2%; Stearic acid (18:0) 5-20%; Oleic acid (18:1) 5-38%; Linoleic acid (18:2) 4-15%; Linolenic acid (18:3) 1-5%; Arachidic acid (20:0) 0-1%; Eicosatrienoic acid (20:3) 1-5%; **Arachidonic acid (20:4) (ARA) 38-44%;** Behenic acid (22:0) 0-3%; Docosapentaenoic acid (22:5) 0-3%; and Lignoceric acid (24:0) 0-3%.

**TABLE 1**

| n-3 and n-6 Fatty Acid Content of Rat Diets | | | | |
|---|---|---|---|---|
| Fatty Acid | Dietary Oil Tested grams fatty acid/100 gram diet | | | |
| | Corn/Soy (BLUE) Comparative | DHASCO^{®} (YELLOW) Comparative | DHA™-S (GREEN) | DHASCO^{®}/ARASCO^{®} (RED) Comparative |
| **n-6 series** | | | | |
| 18:2 linoleic acid | 2.34 | 1.28 | 0.68 | 0.84 |
| 20:4 ARA | 0 | 0 | 0.03 | 0.48 |
| 22:5 DPA | 0 | 0.01 | 0.51 | 0.01 |
| | | | | |
| **n-3 series** | | | | |
| 18:3 linolenic acid | 0.23 | 0.01 | 0.01 | 0.05 |
| 20:5 EPA | 0 | 0 | 0.08 | 0 |
| 22:6 DHA | 0 | 1.27 | 1.25 | 1.27 |
| | | | | |
| % SAT | 27.4% | 27.3% | 26.4% | 26.7% |
| % MONO | 21.0% | 20.8% | 20.1% | 19.9% |
| % POLY | 51.6% | 51.9% | 53.5% | 53.3% |
| n-6 to n-3 ratio | 10.10 | 1.00 | 0.91 | 1.01 |

**TABLE 2**

| Composition of the Oil Mixture used for the Experimental Diets | | | | |
|---|---|---|---|---|
| | | Comparative | Invention | Comparative |
| | Control Diet | DHASCO^{®} | DHA™-S | ARASCO^{®}/DHASCO^{®} |
| | Corn/Soy | DHA | DHA + DPA | ARA + DHA |
| OIL TYPE | g/100g diet | g/100g diet | g/100g diet | g/100g diet |
| DHASCO^{®} | 0 | 30 | 0 | 30 |
| Corn | 15 | 9 | 0 | 0 |
| Soybean | 27 | 0 | 0 | 2 |
| Safflower | 0 | 9 | 8 | 8 |
| Coconut | 8 | 2 | 1 | 0 |
| DHA™-S | 0 | 0 | 31 | 0 |
| ARASCO^{®} | 0 | 0 | 0 | 11 |
| SUNFLOWER | 0 | 0 | 10 | 0 |
| sum | 50 | 50 | 50 | 51 |

All diets contained 50 g of fat/100 g chow. Each of the DHA-containing diets had 1.3 g of DHA/100 g chow and had approximately a 1:1 ratio of n-6 to n-3 fatty acids, compared to the control diet which had approximately a 10:1 ratio of n-6 to n-3 fatty acids. The percent of saturated, monounsaturated and polyunsaturated fatty acids were equivalent across the diets. The amount of total protein (20%) and carbohydrate (66%) as well as total energy (3.9 kcal/gm) were also equivalent across all diets.

### ELISAs and Immunoblot

Aβ ELISAs were performed essentially as described previously (Suzuki et al., Science 264, 1336-1340 (1994). For immunoblot, brains from transgenic and control mice were dounce homogenized in a solution of 2% SDS in H2O containing 0.7 mg/ml Pepstatin A supplemented with complete Mini Protease inhibitor tablet (Roche 1836153). The homogenized mixes were briefly sonicated to shear the DNA and centrifuged at 4°C for 1 hour at 100,000 x g. The supernatant was used for immunoblot analysis. Proteins were resolved by SDS/PAGE (10% Bis-Tris from Invitrogen) under reducing conditions and transferred to nitrocellulose membrane. The membrane was incubated in a 5% solution of nonfat milk for 1 hour at 20°C. After overnight incubation at 4°C with the primary antibody, the blots were washed in Tween-TBS for 20 minutes and incubated at 20°C with the secondary antibody. The blots were washed in T-TBS for 20 minutes and incubated at 20°C with the secondary antibody. The blots were washed in T-TBS for 20 minutes and incubated for 5 minutes with Super Signal (Pierce).

### Biochemical Markers

*Aβ measurements:* quantitative data on the effects of DHA on various species of Aβ (e.g. Aβ40 versus Aβ42; soluble versus insoluble Aβ) (Oddo et al., 2003) was obtained. Protein extracted from brain tissue from mice treated with DHA was used to generate soluble and insoluble protein extracts and analyzed by sandwich ELISA. Western blots were used to measure steady state levels of the APP holoprotein, C99/C83 fragments, and sAPPα to determine the effects of DHA on these biomarkers.

*Tau hyperphosphorylation*: Because the 3xTg-AD mice accumulate argyrophilic and filamentous tau immunoreactive neuronal inclusions with increasing age in the cortex and hippocampus (Oddo et al., 2003), the effects of DHA on tau hyperphosphorylation was measured as a functional biomarker. This was accomplished with quantitative Western blotting with antibodies (such as AT8, AT100, or PHF1) that specifically recognize hyperphosphorylated tau.

Brains were dissected into cortex, hippocampus and cerebellum.

### Immunohistochemistry

To assess total plaques and tangles and also microglial activation, formalin-fixed, parafiin-embedded brains were sectioned at 5 µm, mounted onto silane-coated slides and processed as described. Using various antibodies against various forms of Aβ (1-40, 1-42 and oligomeric) and phosphorylated forms of tau, plaques and tangles were visualized for location and severity within the brain. In addition antibodies such as CD45 were used to stain for microglial activation to determine if plaques and tangles still initiate an immune response. The following antibodies were used: anti- Aβ 6E10 and 4G8 (Signet Laboratories, Dedham, MA), anti- Aβ 1560 (Chemicon), A11 (Kayed et al, 2003), anti-APP 22C11 (Chemicon), anti-Tau HT7, AT8, AT180 (Innogenetics), Tau C17 (Santa Cruz), Tau 5 (Calbiochem), anti-GFAP (Dako) and anti-actin (Sigma). Primary antibodies were applied at dilutions of 1:3000 for GFAP; 1:1000 for 6E10; 1:500 for 1560, AT8, AT180, and Tau5; and 1:200 for HT7.

### Brain Total Lipids Extraction

Brains were maintained at -80°C until analysis. The brains were freeze-dried and the lipids were extracted in 4 mls of 2:1 (v/v) chloroform:methanol with 0.5% BHT as an antioxidant. The mixture was sonicated for 10 minutes and centrifuged to pellet out the solids.

### Fatty Acid Analysis

### Total Brain Lipid Analysis

1.2 mgs of the brain lipid extract were analyzed for brain total fatty acids. The brain total lipids were converted to fatty acid methyl esters (FAME) with 14% BF3/methanol at 100°C for 30 minutes (Morrison, W.R. and Smith, L.M. (1964) J Lipid Res. 5:600-8). Butylated hydroxytoluene was added before saponification and all samples were purged with N2 throughout the process to minimize oxidation. Tricosanoic free fatty acid (23:0) was added to each sample as an internal standard before FAME analysis.

### Brain Phospholipid Analysis

Brain phosphatidylcholine (PC), phosphatidylserine (PS), and phosphatidylethanolamine (PE) were separated using the methods of Gilfillan et al (Gilfillan et al (1983), J Lipid Res. 24: 1651-1656). 0.25 mm thick 20 X 20 cm K silica gel plates (Whatman, Clifton, NJ) were activated for 60 minutes in a 100° C oven. A sample (0.6 mg) of total brain extract was spotted on the plate and developed in a TLC chamber using chloroform:methanol:petroleum ether:acetic acid:boric acid 40:20:30:10:1.8 (v/v/v/v/w). The plate was developed to within 1 cm of the top of the plate. The plate was sprayed with cupric acetate to visualize the bands. The PC, PS and PE bands were scraped into test tubes and the lipids were converted to fatty acid methyl esters (FAME) with 14% BF3/methanol at 100°C for 30 minutes (Morrison and Smith, 1964, *supra*). Butylated hydroxytoluene was added before saponification and all samples were purged with N2 throughout the process to minimize oxidation. Tricosanoic free fatty acid (23:0) was added to each sample as an internal standard before FAME analysis.

### Red Blood Cell Analysis

Total lipids were extracted from 400µl packed red blood cells (RBCs) using the methods of Bligh and Dyer (Bligh, E.G. and Dyer, W.J. (1959), Can. J. Biochem. Physiol. 37:911). Tricosanoic free fatty acid (23:0) was added to each sample as an internal standard before extraction. The RBC lipids were saponified with 0.5 N methanolic sodium hydroxide and the fatty acids were converted to methyl esters with 14% BF3/methanol at 100°C for 30 minutes (Morrison and Smith, 1964, *supra).* Butylated hydroxytoluene was added before saponification and all samples were purged with N2 throughout the process to minimize oxidation.

### Gas Chromatogram Analysis

Fatty acid methyl esters (FAMEs) were analyzed by GLC using a Hewlett Packard 6890 equipped with a flame ionization detector. The fatty acid methyl esters were separated on a 30 meter FAMEWAX capillary column (Restek, Bellefonte, PA; 0.25 mm diameter, 0.25 mm coating thickness) using helium at a flow rate of 2.1 mL/min with split ratios of 48:1 and 20:1. The chromatographic run parameters included an oven starting temperature of 130°C that was increased at 6°C/min to 225°C, where it was held for 20 minutes before increasing to 250°C at 15°C/min, with a final hold of 5 minutes. The injector and detector temperatures were constant at 220°C and 230°C respectively. Peaks were identified by comparison of retention times with external fatty acid methyl ester standard mixtures from NuCheck Prep (Elysian, MN, U.S.A). The fatty acid profiles were expressed as a percent of the total mg of fatty acid (weight percent).

### Example 1

The following example describes the evaluation of the potential of dietary polyunsaturated fatty acids (PUFAs), docosahexaenoic acid (DHA; 22:6 n-3), docosapentaenoic acid (DPAn-6; 22:5 n-6), or arachidonic acid (ARA; 20:4 n-6) to modulate onset or severity of pathophysiological symptoms of disease in a novel triple transgenic mouse model of Alzheimer's Disease (3xTg-AD).

Groups of homozygous 3x-Tg-AD mice were fed one of four diets containing DHA, DHA and DPAn-6, DHA and ARA, or a diet deficient in these PUFAs (control), as described above in the Materials and Methods (see Tables 1 and 2). The three experimental diets contained similar amounts of DHA, and linoleic acid, DPAn-6 or ARA as the n-6 source, as discussed above (see discussion of diets in Materials and Methods). The therapeutic benefits of the control and PUFA-supplemented diets were assessed after several time points of treatment by looking at pathological development in these mice.

Specifically, beginning at age 3 months, the 3xTg-AD mice were fed the prescribed diets as shown in Tables 1 and 2, for up to 12 months of age. Each diet contained reference numbers and was color-coded without information about the level of experimental compound contained in the diet. Stored diets were maintained at 0°C throughout the study. The initiation of the experiment in 3-month old animals was selected to avoid interfering with normal growth and development of the mice while treating with experimental diets before the emergence of Aβ and tau neuropathology. Treatments were stopped at 6, 9, or 12 months of life and a variety of neuropathological assessments on brain and blood were performed, including total APP, amyloid beta peptides, plaque number, plaque size, and levels of APP cleaving enzymes (alpha and beta secretases, presenilin-1). Brain and blood total fatty acids and brain phospholipids were also measured.

At each time point, complete neuropathological, and immunohistochemical analyses were completed on brain and cerebrospinal fluid (CSF) from at least 6 mice to provide for valid statistical analysis. Blood was collected and processed into red blood cell pellets and serum, and stored at -80 C. An additional six mice from each dietary group were sacrificed and blood, and brain sections were shipped frozen for additional analyses. Neuropathology and immunohistochemistry were assessed at 3 time points, when the mice were 6, 9, and 12 months of age.

Table 3 shows the mean body weights after 3 months, 6 months, and 9 months of being fed the PUFA-containing diets described above.

**TABLE 3**

| **Mean body weights after 3 months diet** | | | | |
|---|---|---|---|---|
| | **Male** | **+/-** | **Female** | **+/-** |
| **Blue** | 38.50 | 1.88 | 29.00 | 2.32 |
| **Yellow** | 32.08 | 0.68 | 28.43 | 0.33 |
| **Green** | 40.54 | 2.31 | 32.10 | 1.42 |
| **Red** | 31.23 | 1.23 | 27.43 | 0.73 |
| | | | | |

| **Mean body weights after 6 months diet** | | | | |
|---|---|---|---|---|
| | **Males** | **+/-** | **Females** | **+/-** |
| **Blue** | 39.60 | 5.42 | 26.46 | 0.81 |
| **Yellow** | 47-10 | 5.27 | 32.50 | 1.79 |
| **Green** | 50.13 | 1.74 | 34.53 | 4.22 |
| **Red** | 39.26 | 3.77 | 32.83 | 2.82 |
| | | | | |

| **Mean body weights after 9 months diet** | | | | |
|---|---|---|---|---|
| | **Males** | **+/-** | **Females** | **+/-** |
| **Blue** | 39.68 | 1.688 | 35.40 | 3.15 |
| **Yellow** | 45.81 | 6.16 | 46.44 | 3.95 |
| **Green** | 46.56 | 1.69 | 37.53 | 6.08 |
| **Red** | 47.51 | 2.89 | 32.45 | 0 |

As shown in Table 3, mean body weights of male and female mice did not differ across the diets at 3, 6 or 9 months of treatment. Mice continued to grow and remained healthy on all diets over the course of the study.

### Fatty Acid Analysis Results

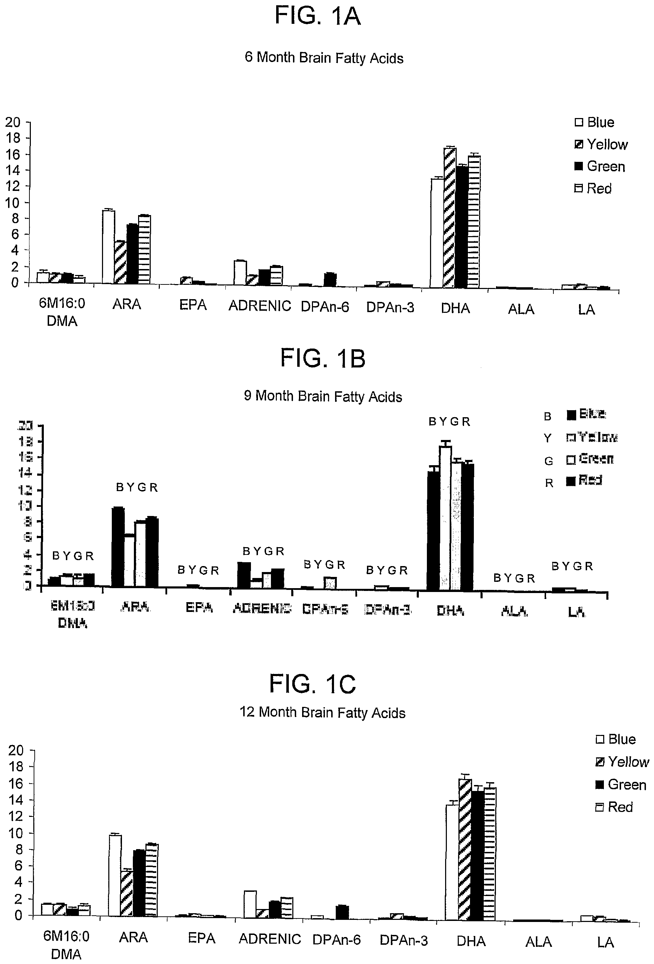

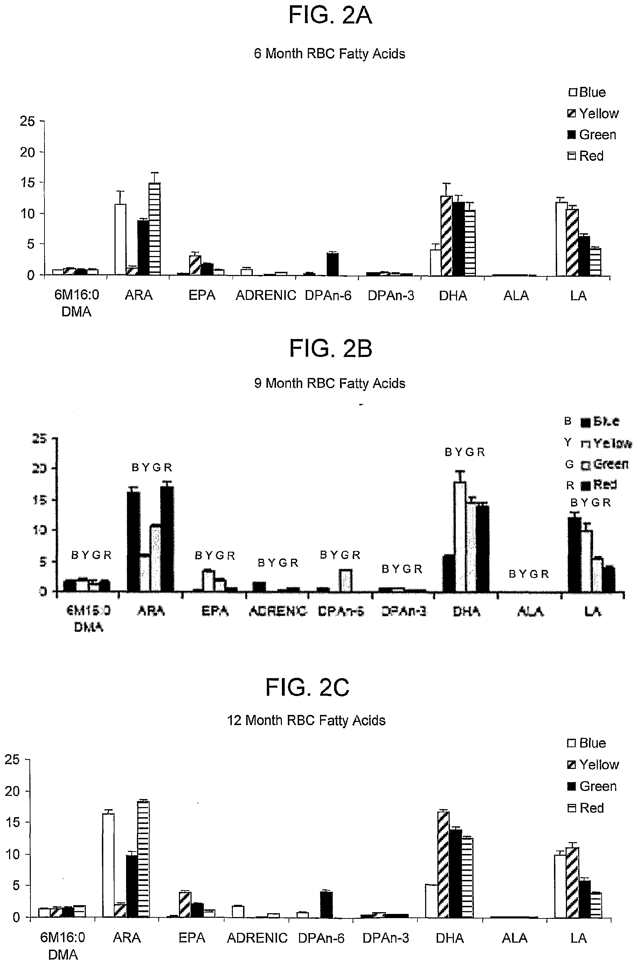

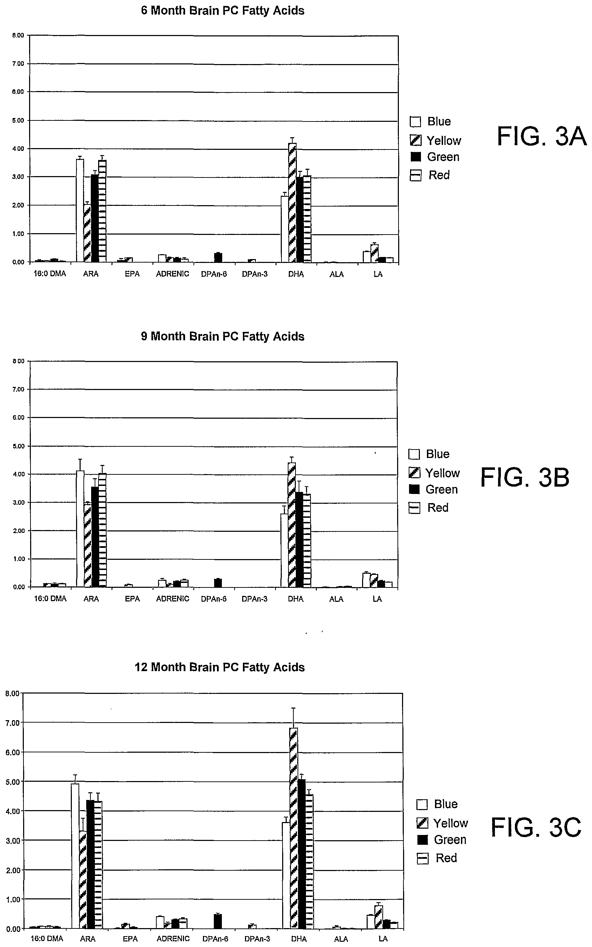

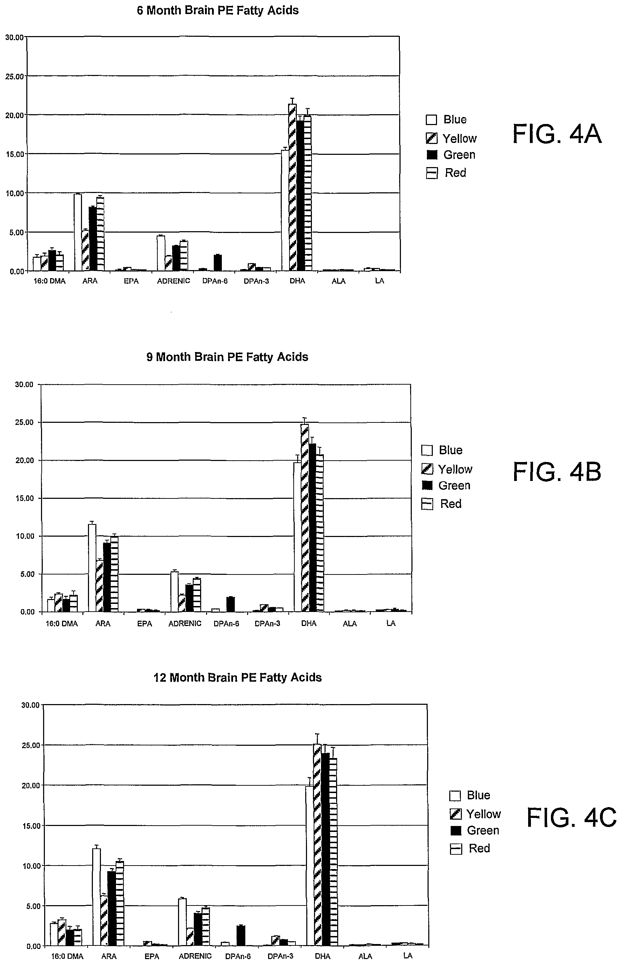

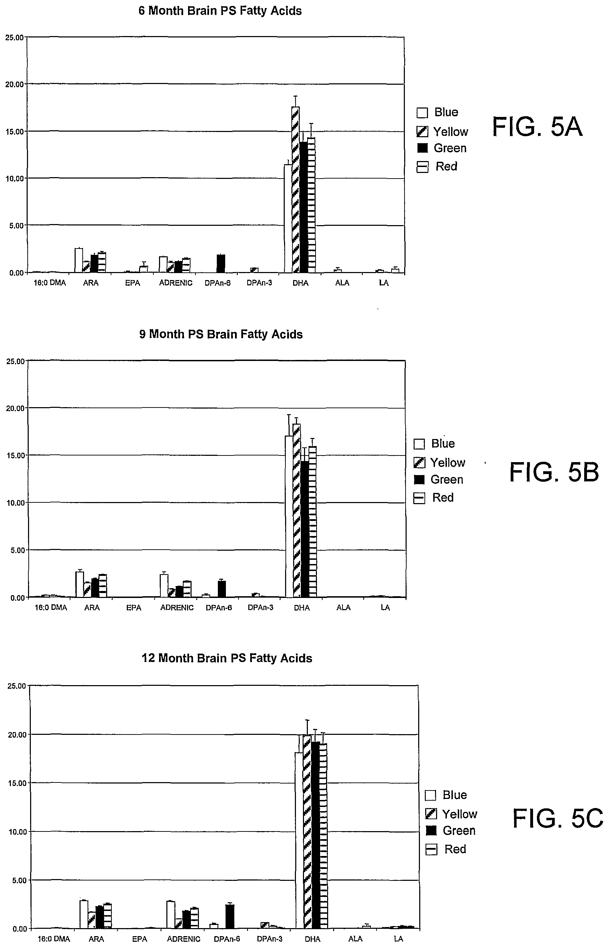

Figs. 1A-C show whole brain homogenate fatty acid profiles of the four dietary treatment groups after 3 months (Fig. 1A; n=6), 6 months (Fig. 1B; n=6), or 9 months (Fig. 1C; n=6) of dietary treatment (values are expressed as a percentage of total brain fatty acids). Figs. 2A-2C show the red blood cell homogenate fatty acid profiles of the four dietary treatment groups after 3 months (Fig. 2A), 6 months (Fig. 2B), or 9 months (Fig. 2C) (values are expressed as a percentage of total red blood cell fatty acids). Figs. 3A-3C show the brain phosphatidylcholine (PC) profiles of the four dietary treatment groups after 3 months (Fig. 3A), 6 months (Fig. 3B), or 9 months (Fig. 3C) (values are expressed as a percentage of total brain PC fatty acids). Figs. 4A-4C show the brain phosphatidylethanolamine (PE) profiles of the four dietary treatment groups after 3 months (Fig. 4A), 6 months (Fig. 4B), or 9 months (Fig. 4C) (values are expressed as a percentage of total brain PE fatty acids). Figs. 5A-5C show the brain phosphatidylserine (PS) profiles of the four dietary treatment groups after 3 months (Fig. 5A), 6 months (Fig. 5B), or 9 months (Fig. 5C) (values are expressed as a percentage of total brain PS fatty acids). In each of Figs. 1-5, the four diets are shown as Control (Blue), DHA (Yellow, comparative example), DHA/DPA (Green) and DHA/ARA (comparative example) (Red). In each of Figs. 1-5, DMA = dimethylacetals; ARA=arachidonic acid (n-6); DHA = docosahexaenoic acid (n-3); EPA= Eicosapentaenoic acid (n-3); LA= linoleic acid (n-6); ALA = alpha-linolenic acid (n-3); DPAn-6 = docosapentaenoic acid (n-6); DPA n-3 = docosapentaenoic acid (n-3); and Adrenic = adrenic acid (n-6).

The results showed that red blood cell (RBC) and brain fatty acids were altered in the 6 month (3 months of dietary treatment), 9 month (6 months of dietary treatment) and 12 month (9 months of dietary treatment) old mice as a result of the PUFA enriched diets (Figs. 1A-C and 2A-C). RBC 22:6 n-3 (DHA) weight percent levels were more than double the control levels with all of the PUFA-supplemented diets at all time points. The total brain 22:6 n-3 (DHA) levels were also increased 1 to 3 weight percent across all PUFA-supplemented diets but not as great as in the RBCs. Mice fed the DHA (yellow) diet had the largest changes in 22:6 n-3 (DHA) weight percents in both the RBCs and the brain total lipids. As a weight percent of fatty acids, 22:6 n-3 (DHA) and 20:4 n-6 (ARA) are the most abundant long chain PUFAs in both the brain and RBC total lipids. 20:5 n-3 (EPA) levels in the brain and RBCs are low and typically, 22:6 n-3 (DHA) weight percent levels in the brain are about fifteen times higher than 20:5 n-3 (EPA), and about five times higher in the RBCs.

While DHA levels increased, there was a subsequent decrease in total brain lipid 20:4 n-6 (ARA) with all three PUFA enriched diets compared to the control diet (which had an n-6 to n-3 ratio of 10:1). Both DHA and ARA brain fatty acid levels were maintained throughout the supplemented period. In the RBCs, the DHA (yellow) diet greatly lowered RBC 20:4 n-6 (ARA) levels by 11.75 weight percent compared to the control group across all time points. As expected, the mice on the red diet (DHA- and ARA-supplemented) had 20:4 n-6 (ARA) RBC levels closest to the control mice.

22:5 n-6 (DPAn-6) levels in the RBCs and total brain lipids were more than double the control levels in the DHA and DPAn-6 (green) diet across all time points. Brain and RBC 22:5 n-6 (DPA) levels were very low or non-detectable in the mice fed the DHA (yellow) or DHA and ARA (red) diets. There is minimal 18:3 n-3 (ALA) in the brain and RBC lipids and very small fatty acid changes were seen in either tissue type for this precursor of long-chain PUFAs. 18:2 n-6 (LA) levels are about fifteen times higher in RBCs compared to brain. RBC 18:2 n-6 (LA) weight percent levels were lower than the controls with all PUFA enriched diets across the time points. These changes are reflective of the fatty acid compositions of the diets administered. RBC 20:5 n-3 (EPA) weight percent levels were higher across all PUFA supplemented diets vs. control levels, even though these diets did not contain appreciable amounts of 20:5 n-3 (EPA). This may signify the retroconversion of 22:6 n-3 (DHA) to 20:5 n-3 (EPA) in blood cells. No appreciable amounts of 20:5 n-3 (EPA) were detected in brain lipids across the various PUFA supplemented diets and time periods.

PS, PE and the PC brain phospholipid fatty acids were also altered in the 6 month (3 months of dietary treatment), 9 month (6 months of dietary treatment) and 12 month (9 months of dietary treatment) old mice with the PUFA enriched diets (see Figs. 3A-3C, 4A-4C and 5A-5C). As a weight percent, 22:6 n-3 (DHA) is 5 times more abundant in the PS (Fig. 5) and PE (Fig. 4) fractions compared to the PC (Fig. 3) fraction, and 20:4 n-6 (ARA) is most abundant in the PE fraction. 20:5 n-3 (EPA), 18:2 n-6 (LA) and 18:3 n-3 (ALA) levels in the brain phospholipids are very low. The DHA (yellow) diet led to the largest increase from control in the brain phospholipid 22:6 n-3 (DHA) weight percents for all three phospholipids. There was also a corresponding decrease in 20:4 n-6 (ARA) levels with the DHA (yellow) diet compared to controls in all three brain phospholipids. As seen in the RBCs and total brain lipids, the 22:5 n-6 (DPA) weight percent levels in all brain phospholipid fractions increased most in the mice fed the DHA- and DPAn-6-enriched (green) diet.

In summary, the ratios of n-6 to n-3 fatty acid compositions of the diets were reflected in RBC and brain fatty acid levels. Increasing 22:6 n-3 (DHA) content in the diets led to significant increases in DHA levels in both RBC and brain levels. With increased levels of 22:6 n-3 (DHA) in the diet, there was a subsequent decrease in n-6 fatty acids. When other fatty acids (i.e. DPA and ARA) were added to the diets, their corresponding fatty acid levels increased in the tissues as well. Overall, brain fatty acid levels were well-maintained throughout the supplementation period for each diet.

### Biochemical Marker Analysis

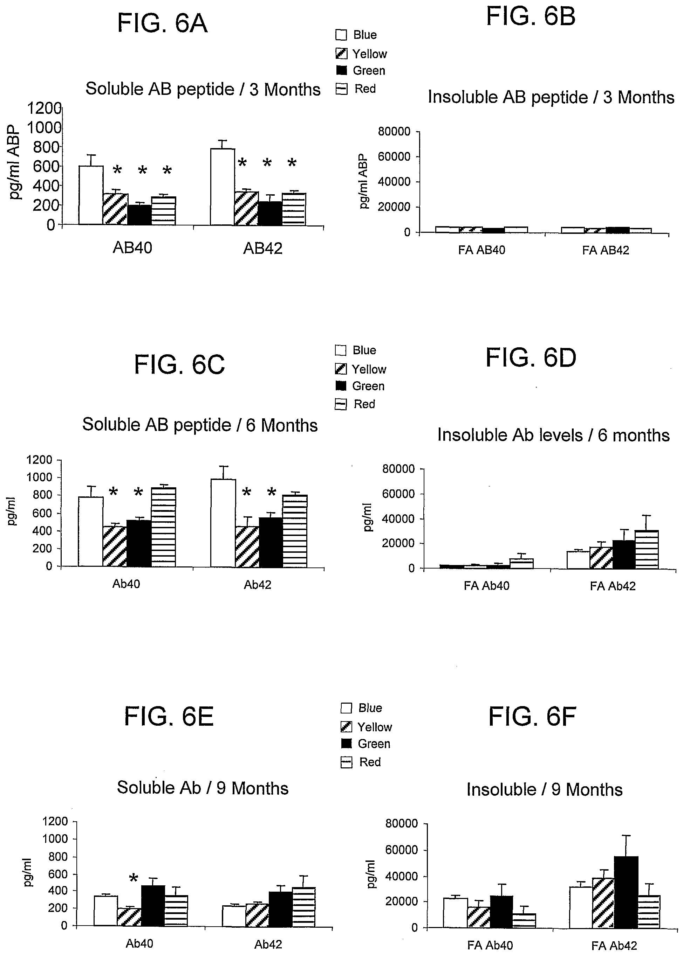

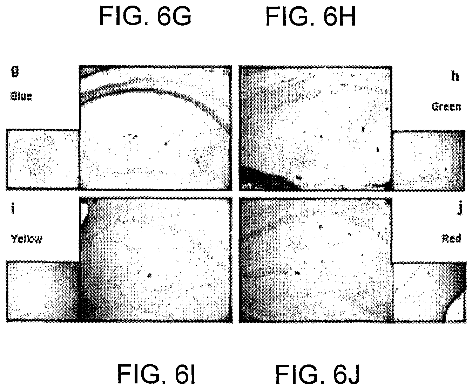

Figs. 6A-6J show the effect of diets on Amyloid-β (Aβ) levels in 6-month-old 3X-TG-AD mice after 3 months, 6 months and 9 months of dietary treatment. As described above, animals received one of the four diets shown in Table 1 above. Total Soluble (Figs. 6A, 6C and 6E) and insoluble (Figs. 6B, 6D and 6F) Aβ peptide were measured from brain protein extract with antibodies specific to Aβ 1-40 and Aβ 1-42 and total amyloid. As shown in Fig. 6A, after 3 months of feeding, animals fed diets containing microalgal DHA (yellow, green and red diets) had significantly lower levels of soluble amyloid beta peptides compared to animals fed corn-soy oil. At 6 months of feeding the dietary supplements (Fig. 6C), animals fed diets containing DHA (yellow) or DHA and DPAn-6 (green) still had significantly lower levels of amyloid beta peptides compared to control animals, but the level of amyloid beta peptide in animals fed a diet containing DHA and ARA (red) was no longer significantly different than controls. At 9 months of feeding (Fig. 6E), only animals fed a diet containing DHA (yellow) had significantly reduced Aβ as compared to the control. No differences in total levels of insoluble amyloid were observed between diets containing corn/soy and microalgal oils. Figs. 6G-6J show relative intensity of intracellular total amyloid present in coronal sections from animals fed corn/soy (blue group), DHASCO^{®} (yellow group), DHA™-S (green group), and DHASCO^{®}/ARASCO^{®} (red group). Animals fed corn-soy and DHASCO^{®}/ARASCO^{®} diets contained relatively more total intracellular amyloid than animals fed the DHASCO^{®} or DHA™-S containing diets after 3 months of feeding.

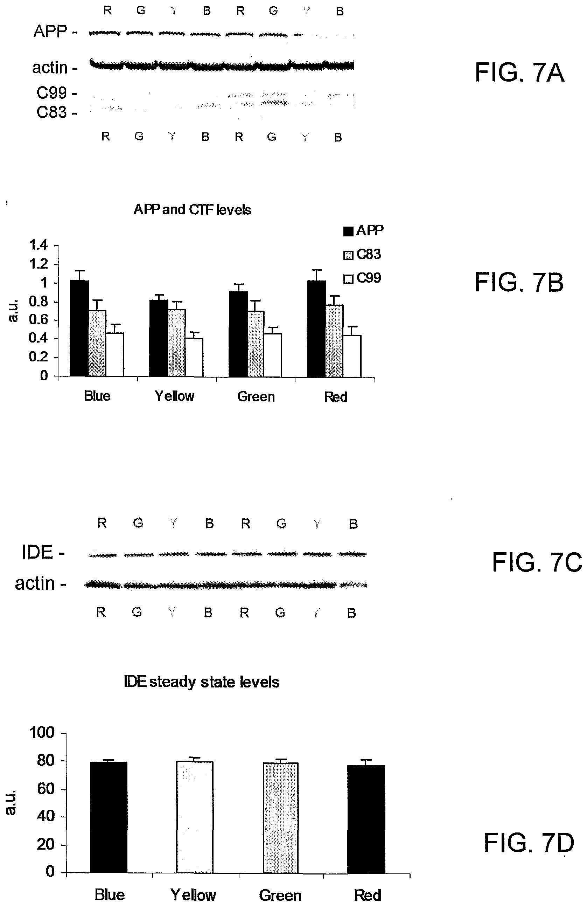

Figs. 7A and 7B show the effects of diets on APP processing in 6 month old 3x-TG AD mice after 3 months of treatment. Total levels of APP, C83 and C99 did not differ significantly between animals fed diets containing corn-soy oil or microalgal oils.

Figs. 7C and 7D show the effects of diets on Aβ peptide clearance enzyme (insulin degrading enzyme, IDE) after 3 months of treatment. No statistically significant differences in IDE levels were observed in animals fed diets containing corn/soy or various microalgal oils.

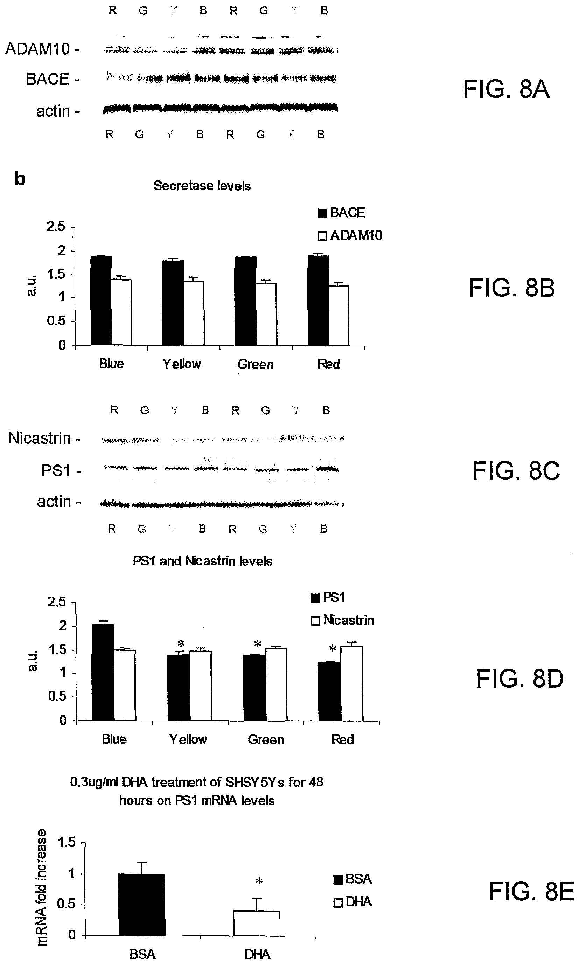

Figs. 8A and 8B show the effects of diets on APP secretases in 6 month old 3X-TG animals after 3 months of experimental dietary treatment. No significant differences in β-secretase (BACE) or ADAM 10 were evident in animals fed diets containing corn-soy or microalgal oils.

Figs. 8C and 8D show that, as compared to animals fed corn-soy diets, animals fed diets containing microalgal oils significantly had reduced levels of the enzyme, presenilin 1, but not nicastrin.

Fig. 8E shows that DHA significantly reduced presenilin 1 mRNA (*, p < 0.05) in SHSY5Y cells.

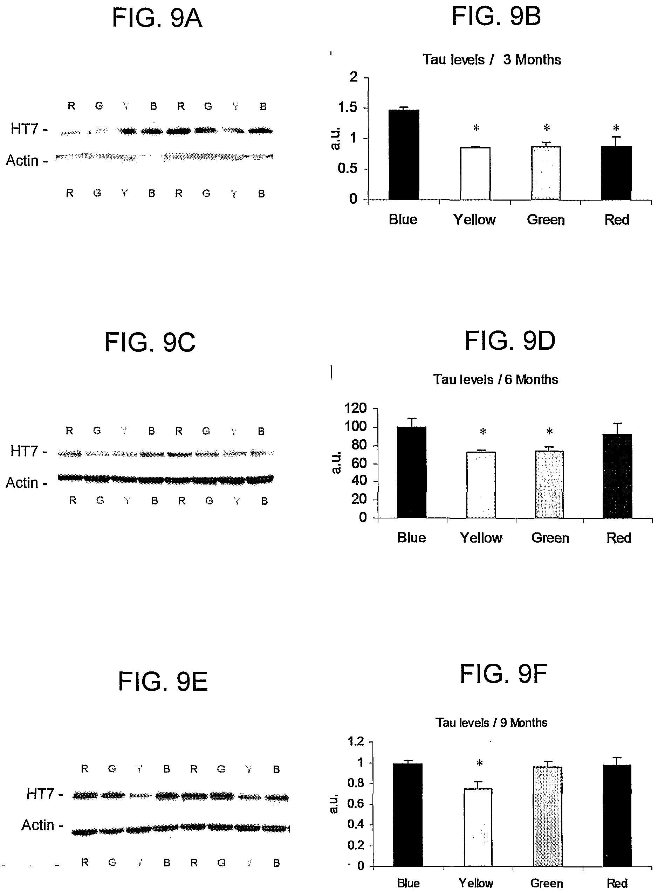

Figs. 9A-9F show the effects of diets on total tau levels after 3 months, 6 months and 9 months of dietary treatment. As shown in Figs. 9A and 9B, diets containing DHA-containing microalgal oils (yellow, green and red diets) significantly reduced total tau levels compared to diets containing corn-soy oil. After 6 months of feeding, animals fed diets containing DHA as the predominant PUFA (yellow) or DHA and DPAn-6 (green) retained significantly lower total tau levels compared to diets containing corn-soy oil, but animals fed diets containing a combination of DHA and ARA (red) did not have significantly different total tau levels as compared to controls. After 9 months of feeding, only animals fed diets containing DHA as the predominant PUFA (yellow) had significantly reduced total tau levels as compared to controls.

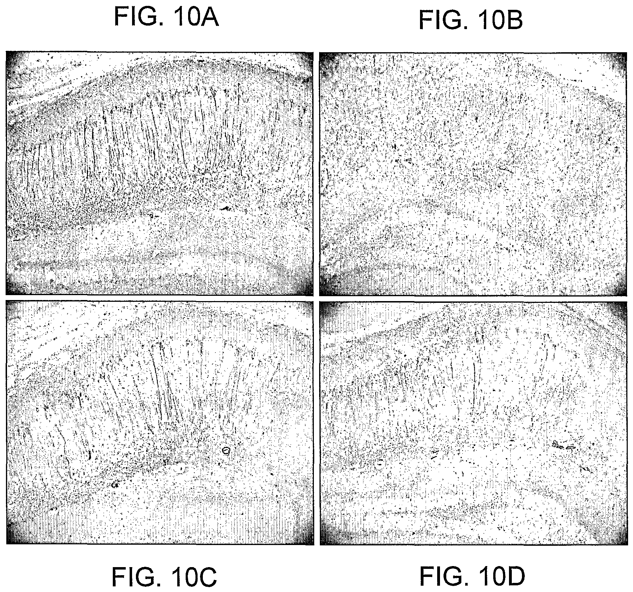

Figs. 10A-10D show that the DHA and DPAn-6 (green) diet significantly reduced the expression of conformationally altered tau compared to the other 3 diets after 3 months of feeding.

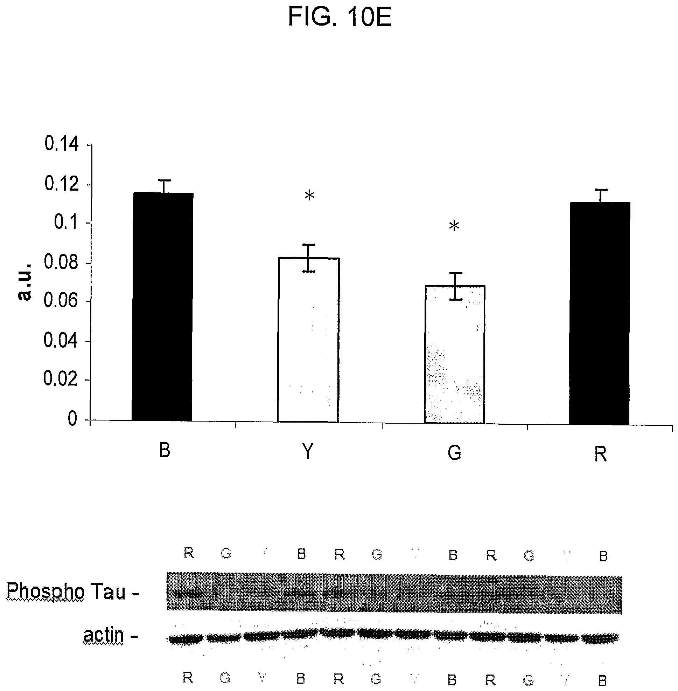

As shown in Fig. 10E and 10F, after 9 months of dietary treatment, phosphorylated tau is significantly reduced in animals fed diets containing DHA as the predominant PUFA (yellow) or DHA and DPAn-6 (green), as compared to animals fed diets containing corn-soy oil. Animals fed diets containing a combination of DHA and ARA (red) did not have significantly different phosphorylated tau levels as compared to controls.
(i.e. DPA and ARA) were added to the diets, their corresponding fatty acid levels increased in the tissues as well. Overall, brain fatty acid levels were well-maintained throughout the supplementation period for each diet.

### Biochemical Marker Analysis

Figs. 6A-6J show the effect of diets on Amyloid-β (Aβ) levels in 6-month-old 3X-TG-AD mice after 3 months, 6 months and 9 months of dietary treatment. As described above, animals received one of the four diets shown in Table 1 above. Total Soluble (Figs. 6A, 6C and 6E) and insoluble (Figs. 6B, 6D and 6F) Aβ peptide were measured from brain protein extract with antibodies specific to Aβ 1-40 and Aβ 1-42 and total amyloid. As shown in Fig. 6A, after 3 months of feeding, animals fed diets containing microalgal DHA (yellow, green and red diets) had significantly lower levels of soluble amyloid beta peptides compared to animals fed corn-soy oil. At 6 months of feeding the dietary supplements (Fig. 6C), animals fed diets containing DHA (yellow) or DHA and DPAn-6 (green) still had significantly lower levels of amyloid beta peptides compared to control animals, but the level of amyloid beta peptide in animals fed a diet containing DHA and ARA (red) was no longer significantly different than controls. At 9 months of feeding (Fig. 6E), only animals fed a diet containing DHA (yellow) had significantly reduced Aβ as compared to the control. No differences in total levels of insoluble amyloid were observed between diets containing corn/soy and microalgal oils. Figs. 6G-6J show relative intensity of intracellular total amyloid present in coronal sections from animals fed corn/soy (blue group), DHASCO^{®} (yellow group), DHA^{™}-S (green group), and DHASCO^{®}/ARASCO^{®} (red group). Animals fed corn-soy and DHASCO^{®}/ARASCO^{®} diets contained relatively more total intracellular amyloid than animals fed the DHASCO^{®} or DHA^{™}-S containing diets after 3 months of feeding.

Figs. 7A and 7B show the effects of diets on APP processing in 6 month old 3x-TG AD mice after 3 months of treatment. Total levels of APP, C83 and C99 did not differ significantly between animals fed diets containing corn-soy oil or microalgal oils.

Figs. 7C and 7D show the effects of diets on Aβ peptide clearance enzyme (insulin degrading enzyme, IDE) after 3 months of treatment. No statistically significant differences in IDE levels were observed in animals fed diets containing corn/soy or various microalgal oils.

Figs. 8A and 8B show the effects of diets on APP secretases in 6 month old 3X-TG animals after 3 months of experimental dietary treatment. No significant differences in β-secretase (BACE) or ADAM 10 were evident in animals fed diets containing corn-soy or microalgal oils.

Figs. 8C and 8D show that, as compared to animals fed corn-soy diets, animals fed diets containing microalgal oils significantly had reduced levels of the enzyme, presenilin 1, but not nicastrin.

Fig. 8E shows that DHA significantly reduced presenilin 1 mRNA (*, p < 0.05) in SHSY5Y cells.

Figs. 9A-9F show the effects of diets on total tau levels after 3 months, 6 months and 9 months of dietary treatment. As shown in Figs. 9A and 9B, diets containing DHA-containing microalgal oils (yellow, green and red diets) significantly reduced total tau levels compared to diets containing corn-soy oil. After 6 months of feeding, animals fed diets containing DHA as the predominant PUFA (yellow) or DHA and DPAn-6 (green) retained significantly lower total tau levels compared to diets containing corn-soy oil, but animals fed diets containing a combination of DHA and ARA (red) did not have significantly different total tau levels as compared to controls. After 9 months of feeding, only animals fed diets containing DHA as the predominant PUFA (yellow) had significantly reduced total tau levels as compared to controls.

Figs. 10A-10D show that the DHA and DPAn-6 (green) diet significantly reduced the expression of conformationally altered tau compared to the other 3 diets after 3 months of feeding.

As shown in Fig. 10E and 10F, after 9 months of dietary treatment, phosphorylated tau is significantly reduced in animals fed diets containing DHA as the predominant PUFA (yellow) or DHA and DPAn-6 (green), as compared to animals fed diets containing corn-soy oil. Animals fed diets containing a combination of DHA and ARA (red) did not have significantly different phosphorylated tau levels as compared to controls.

Each reference and publication cited herein is incorporated by reference in its entirety. The entire disclosure of each of U.S. Provisional Application No. 60/697,911 and U.S. Provisional Application No. 60/779,145 is incorporated herein by reference.

While various embodiments of the present invention have been described in detail, it is apparent that modifications and adaptations of those embodiments will occur to those skilled in the art. It is to be expressly understood, however, that such modifications and adaptations are within the scope of the present invention, as set forth in the following claims.

## Claims

1. Use of a composition comprising docosahexaenoic acid (DHA) and docosapentaenoic acid n-6 (DPAn-6), wherein the ratio of DHA to DPAn-6 is from 1:1 to 10:1, in the preparation of a medicament formulated for oral administration for treating dementia or pre-dementia.

2. The use as recited in claim 1, wherein the DHA is to be administered in an amount from 50 mg per day to 20 grams per day.

3. The use as recited in claim 1, wherein the oral formulation comprises an oil or formulation comprising 40% or more by weight of a combination of DHA and DPAn-6.

4. The use as recited in claim 1, wherein the oral formulation comprises an oil or formulation comprising 50% or more by weight of a combination of DHA and DPAn-6.

5. The use as recited in claim 1, wherein the oral formulation comprises an oil or formulation comprising 60% or more by weight of a combination of DHA and DPAn-6.

6. The use as recited in claim 1, wherein the oral formulation comprises an oil or formulation comprising 70% or more by weight of a combination of DHA and DPAn-6.

7. The use as recited in claim 1, wherein the oral formulation comprises an oil or formulation comprising 80% or more by weight of a combination of DHA and DPAn-6.

8. The use as recited in claim 1, wherein the DHA is in the form of a triglyceride oil.

9. The use as recited in claim 1, wherein the DHA is in the form of an ester.

10. A pharmaceutical composition comprising docosahexaenoic acid (DHA) and docosapentaenoic acid n-6 (DPAn-6) in a ratio of from 1:1 to 10:1 in an oral dosage for the treatment of dementia or pre-dementia in a human.

11. The composition of claim 10, wherein the amount of DHA administered to said human is from 50 mg to 20 grams per day.

12. The composition of claim 10, wherein the composition comprises an oil or formulation comprising 40% or more by weight of a combination of DHA and DPAn-6.

13. The composition of claim 10, wherein the composition comprises an oil or formulation comprising 50% or more by weight of a combination of DHA and DPAn-6.

14. The composition of claim 10, wherein the composition comprises an oil or formulation comprising 60% or more by weight of a combination of DHA and DPAn-6.

15. The composition of claim 10, wherein the composition comprises an oil or formulation comprising 70% or more by weight of a combination of DHA and DPAn-6.

16. The composition of claim 10, wherein the composition comprises an oil or formulation comprising 80% or more by weight of a combination of DHA and DPAn-6.

17. The composition of claim 10, wherein the DHA is in the form of a triglyceride oil.

18. The composition of claim 10, wherein the DHA is in the form of an ester.

## Patentansprüche

1. Verwendung einer Docosahexaensäure (DHA) und Docosapentaensäure (DPAn-6) umfassenden Zusammensetzung, wobei das Verhältnis von DHA zu DPAn-6 1:1 bis 10:1 beträgt, bei der Herstellung eines zur oralen Verabreichung formulierten Arzneimittels zur Behandlung von Demenz oder Prä-Demenz.

2. Verwendung gemäß Anspruch 1, wobei die DHA in einer Menge von 50 mg pro Tag bis 20 Gramm pro Tag verabreicht werden soll.

3. Verwendung gemäß Anspruch 1, wobei die orale Formulierung ein Öl bzw. eine Formulierung umfasst, das bzw. die 40 oder mehr Gew.-% einer Kombination von DHA und DPAn-6 umfasst.

4. Verwendung gemäß Anspruch 1, wobei die orale Formulierung ein Öl bzw. eine Formulierung umfasst, das bzw. die 50 oder mehr Gew.-% einer Kombination von DHA und DPAn-6 umfasst.

5. Verwendung gemäß Anspruch 1, wobei die orale Formulierung ein Öl bzw. eine Formulierung umfasst, das bzw. die 60 oder mehr Gew.-% einer Kombination von DHA und DPAn-6 umfasst.

6. Verwendung gemäß Anspruch 1, wobei die orale Formulierung ein Öl bzw. eine Formulierung umfasst, das bzw. die 70 oder mehr Gew.-% einer Kombination von DHA und DPAn-6 umfasst.

7. Verwendung gemäß Anspruch 1, wobei die orale Formulierung ein Öl bzw. eine Formulierung umfasst, das bzw. die 80 oder mehr Gew.-% einer Kombination von DHA und DPAn-6 umfasst.

8. Verwendung gemäß Anspruch 1, wobei die DHA in Form eines Triglycerid-Öls vorliegt.

9. Verwendung gemäß Anspruch 1, wobei die DHA in Form eines Esters vorliegt.

10. Pharmazeutische Zusammensetzung, umfassend Docosahexaensäure (DHA) und Docosapentaensäure (DPAn-6) in einem Verhältnis von 1:1 bis 10:1 in einer oralen Dosierung für die Behandlung von Demenz oder Prä-Demenz bei einem Menschen.

11. Zusammensetzung nach Anspruch 10, wobei die dem Menschen verabreichte DHA-Menge 50 mg bis 20 Gramm pro Tag beträgt.

12. Zusammensetzung nach Anspruch 10, wobei die Zusammensetzung ein Öl bzw. eine Formulierung umfasst, das bzw. die 40 oder mehr Gew.-% einer Kombination von DHA und DPAn-6 umfasst.

13. Zusammensetzung nach Anspruch 10, wobei die Zusammensetzung ein Öl bzw. eine Formulierung umfasst, das bzw. die 50 oder mehr Gew.-% einer Kombination von DHA und DPAn-6 umfasst.

14. Zusammensetzung nach Anspruch 10, wobei die Zusammensetzung ein Öl bzw. eine Formulierung umfasst, das bzw. die 60 oder mehr Gew.-% einer Kombination von DHA und DPAn-6 umfasst.

15. Zusammensetzung nach Anspruch 10, wobei die Zusammensetzung ein Öl bzw. eine Formulierung umfasst, das bzw. die 70 oder mehr Gew.-% einer Kombination von DHA und DPAn-6 umfasst.

16. Zusammensetzung nach Anspruch 10, wobei die Zusammensetzung ein Öl bzw. eine Formulierung umfasst, das bzw. die 80 oder mehr Gew.-% einer Kombination von DHA und DPAn-6 umfasst.

17. Zusammensetzung nach Anspruch 10, wobei die DHA in Form eines Triglycerid-Öls vorliegt.

18. Zusammensetzung nach Anspruch 10, wobei die DHA in Form eines Esters vorliegt.

## Revendications

1. Utilisation d'une composition comprenant de l'acide docosahexénoïque (DHA) et de l'acide docosapenténoïque n-6 (DPAn-6), où le rapport du DHA sur le DPAn-6 est compris entre 1:1 et 10:1, dans l'élaboration d'un médicament formulé pour administration orale et destiné au traitement de la démence ou de la pré-démence.

2. Utilisation selon la revendication 1, où le DHA est destiné à être administré à une teneur comprise entre 50 mg par jour et 20 grammes par jour.

3. Utilisation selon la revendication 1, où la formule orale comprend une huile ou une formule comprenant 40 % en masse ou plus d'une combinaison de DHA et de DPAn-6.

4. Utilisation selon la revendication 1, où la formule orale comprend une huile ou une formule comprenant 50 % en masse ou plus d'une combinaison de DHA et de DPAn-6.

5. Utilisation selon la revendication 1, où la formule orale comprend une huile ou une formule comprenant 60 % en masse ou plus d'une combinaison de DHA et de DPAn-6.

6. Utilisation selon la revendication 1, où la formule orale comprend une huile ou une formule comprenant 70 % en masse ou plus d'une combinaison de DHA et de DPAn-6.

7. Utilisation selon la revendication 1, où la formule orale comprend une huile ou une formule comprenant 80 % en masse ou plus d'une combinaison de DHA et de DPAn-6.

8. Utilisation selon la revendication 1, où le DHA se présente sous la forme d'une huile de triglycérides.

9. Utilisation selon la revendication 1, où le DHA se présente sous la forme d'un ester.

10. Composition pharmaceutique comprenant de l'acide docosahexénoïque (DHA) et de l'acide docosapenténoïque n-6 (DPAn-6) dans un rapport compris entre 1:1 et 10:1 en dose orale destinée au traitement de la démence ou de la pré-démence chez un humain.

11. Composition selon la revendication 10, où la quantité de DHA administrée audit humain est comprise entre 50 mg et 20 grammes par jour.

12. Composition selon la revendication 10, où la composition comprend une huile ou une formule comprenant 40 % en masse ou plus d'une combinaison de DHA et de DPAn-6.

13. Composition selon la revendication 10, où la composition comprend une huile ou une formule comprenant 50 % en masse ou plus d'une combinaison de DHA et de DPAn-6.

14. Composition selon la revendication 10, où la composition comprend une huile ou une formule comprenant 60 % en masse ou plus d'une combinaison de DHA et de DPAn-6.

15. Composition selon la revendication 10, où la composition comprend une huile ou une formule comprenant 70 % en masse ou plus d'une combinaison de DHA et de DPAn-6.

16. Composition selon la revendication 10, où la composition comprend une huile ou une formule comprenant 80 % en masse ou plus d'une combinaison de DHA et de DPAn-6.

17. Composition selon la revendication 10, où le DHA se présente sous la forme d'une huile de triglycérides.

18. Composition selon la revendication 10, où le DHA se présente sous la forme d'un ester.
